# EUROPEAN PATENT APPLICATION

(11) **EP 4 512 254 A2**
(43) Date of publication of application: **26.02.2025**
(21) Application number: 24217443.1
(22) Date of filing: 16.09.2020
(51) Int. Cl.: A23L 23/00

(54) **METHOD FOR THE CONVERSION OF INSECTS INTO NUTRIENT STREAMS AND PRODUCTS RESULTING THEREFROM**

(30) Priority: 17.09.2019 NL 2023838; 17.12.2019 NL 2024481; 11.05.2020 NL 2025547; 11.05.2020 NL 2025546
(62) Divisional of application: 20775083.7
(71) Applicant: Protix B.V., 5107 NC Dongen (NL)
(72) Inventor: Paul, Aman, 5121 CD Rijen (NL)
(74) Representative: Nederlandsch Octrooibureau

(57) **Abstract**

The present invention relates to a method for converting insects into nutrient streams, such as a puree, an enzymatically hydrolysed puree, a fat fraction and a protein fraction. The invention further relates to the (hydrolysed) puree and to the fractions obtainable by said methods and to their use as a food or feed product or as an ingredient therefore. The invention also relates to a method for converting insect larvae into a composition with anti-oxidant properties. Thus, the invention also relates to a method for converting insect larvae into an antioxidant composition. In addition, the invention relates to a composition with anti-oxidant properties obtainable by said method and to the use of the composition as a health promoting food ingredient or as a feed ingredient.

## Description

### TECHNICAL FIELD OF THE INVENTION

The present invention relates to a method for converting insects into nutrient streams, such as a puree, an enzymatically hydrolysed puree, a fat fraction and a protein fraction. The invention further relates to the (hydrolysed) puree and to the fractions obtainable by said methods and to their use as a food or feed product. The invention also relates to a method for converting insect larvae into a composition with anti-oxidant properties. In addition, the invention relates to a composition with anti-oxidant properties obtainable by said method and to the use of the composition as a health promoting food ingredient or as a feed ingredient.

### BACKGROUND OF THE INVENTION

Urban solid waste management is considered one of the most immediate and serious environmental problems confronting urban governments. The severity of this challenge will increase in the future given the trends of rapid urbanisation and growth in urban population. At present recycling organic waste material is still fairly limited, although this is by far the largest fraction of all generated municipal waste.

A fairly novel approach to bio-waste conversion is by insect larvae, using for example the black soldier fly (*Hermetia illucens*)*,* the house fly (*Musca domestica*)*,* and the meal worm (*Tenebrio molitor L*.). Their popularity links to the promising opportunities of using the harvested larvae as a source of protein and fat for animal feed or food products, thus providing a valuable alternative to other (animal) sources of protein and fat.

Even though many people dislike the consumption of whole insects, the use of insects as a source for separated fats and proteins, which are subsequently used in the preparation of food and feed is more acceptable. However, in order for insects to become a commercially viable nutrient source their production and processing should be carried out on a large scale and the nutrient streams obtained should be free from unwanted flavours and discolorations.

Although large scale insect production facilities are already in use, the economical preparation of nutrient streams of sufficient quality remains a challenge. In the international patent application WO2014123420 a general method has been described for obtaining nutrient streams from insects or worms. Despite the fact that the methods disclosed therein are able to provide nutrient streams of sufficient quality, a need remains for further increasing the yield of such methods and the quality of the nutrient streams obtained therewith.

Insects are commonly consumed as food in many cultures around the world. In European countries, insect proteins are gaining rapid acceptance as high value protein ingredients in animal diets. The European Union has already approved the inclusion of insect proteins in pet food and aquaculture feed formulations. Chicken meal and fish meal are common ingredients in pet food and aquaculture feed preparations, respectively. Insect proteins are increasingly being viewed as an alternative to chicken meal and fish meal in these markets. Amongst all the insect being produced on industrial scale, black soldier fly (*Hermetia illucens*) larvae has gained special attention due to its ability to grow on wide range of organic residues and unique nutritional composition. The nutritional suitability of black soldier fly larvae (BSF) proteins in aquaculture and pet diets is well established.

Pets develop wide range of health disorders with age. Aging can accelerate the free radical damage in a pet's body which might lead to cognitive and locomotor system malfunctioning. Similarly oxidative stress in fishes as a result of immune response could lead to compromised health. Neutrophils (white blood cells) are responsible for the primary defense mechanism of the body. Upon receiving the signal, neutrophils rush to the site of intrusion by pathogenic microbes. Neutrophils then inactivate the pathogens by phagocytosis and release of reactive oxygen species (ROS). Production of ROS is crucial for the host defense. However, in the long term, excessive ROS production by neutrophils could damage animal cells and might lead to cellular aging, cancer, reduced immunity, *etc.* Dietary interventions that can scavenge ROS may help in reducing oxidative damage in the animal body and resulting health conditions. The field is in need of such dietary interventions.

The present invention aims to further improve the yield and quality of the nutrient streams obtained from insects over methods known in the prior art. The invention is further aimed at the nutrient streams obtained with such methods.

### SUMMARY OF THE INVENTION

A first aspect of the present invention relates to a method to convert insects into nutrient streams, comprising the steps of:
a) providing fresh insects and preparing a pulp thereof;
b) heating the pulp for 50-100 seconds at a temperature of 60-95°C
c) subjecting the heated pulp to a physical separation step thereby obtaining a fat fraction, an aqueous protein fraction and a solid-containing fraction.

An aspect of the present invention relates to a method to convert insects into nutrient streams, comprising the steps of:
a) providing fresh insects and preparing a pulp thereof; and
b) heating the pulp for 50-100 seconds at a temperature of 60-95°C,
thereby obtaining an insect puree (pulp). The nutrient streams is an insect puree obtained after the heating of the prepared pulp from fresh insects, such as Black Soldier Fly (BSF, black soldier fly) larvae. Optionally, the method comprises a further step c) after step b): subjecting the heated pulp to a physical separation step thereby obtaining a fat fraction, an aqueous protein fraction and a solid-containing fraction. Drying of the protein fraction reveals protein meal obtained from insect such as BSF larvae.

An aspect of the present invention relates to a method to convert insects into nutrient streams, comprising the steps of:
a) providing fresh insects and preparing a pulp thereof;
a1) subjecting the pulp of step a) to enzymatic hydrolysis by contacting the pulp with a peptidase at a predetermined temperature and for a predetermined duration of time; subsequently
b) heating the hydrolysed pulp of step a1) for 50-100 seconds at a temperature of 60-95°C,
thereby obtaining a hydrolysed insect puree. The nutrient streams is a hydrolysed insect puree obtained after the heating of the hydrolysed pulp, the pulp obtained from fresh insects, such as BSF larvae. Optionally, the method comprises a further step c) after step b): subjecting the heated and hydrolysed pulp to a physical separation step thereby obtaining a fat fraction, an aqueous protein fraction and a solid-containing fraction. Drying of the protein fraction reveals hydrolysed protein meal obtained from insect such as BSF larvae.

It has been found that with the method of the present invention it is possible to increase the yield of fat from the processed insects. It has surprisingly been found that by using the present time-temperature combination less oil is entrapped with the protein fraction. Since the method of the invention results in less protein aggregation and granulate formation in step b) and step c) of the invention, when compared to methods applying heating times for longer than 100 seconds such as at least 5 minutes, less oil can become entrapped at and inside such protein aggregates (without wishing to be bound by any theory). It has also been found that discolouration of the pulp and the nutrient fractions is reduced. The method of the present invention also saves energy as considerably less time is needed to heat the pulp as has previously been suggested in the prior art. Furthermore, the inventors found that the heated insect pulp and the heated hydrolysed insect pulp have anti-oxidant properties.

In an embodiment, the method to convert insects into nutrient streams, comprises the steps of:
a) providing fresh insects and preparing a pulp thereof;
b) heating the pulp for 50-100 seconds at a temperature of 60-95°C
c) subjecting the heated pulp to a physical separation step thereby obtaining a fat fraction, an aqueous protein fraction and a solid-containing fraction and further comprises a step a1) of treating the pulp by an enzyme prior to the step b) (e.g. enzymatic hydrolysis of proteins in the pulp). The pulp might be treated by the enzyme for 0,5 - 3 hours such as 1 to 2 hours at a temperature of 40°C to 70°C, preferably at 45°C to 65°C, more preferably at a temperature of 50°C ± 2°C or about 50°C. The enzyme might be a protease such as a peptidase, preferably the enzyme is Flavourzyme. It has been found out that this enzyme treatment step a1) prior to the heating step b) increases the free amino acid content and digestibility of the obtained insect (larvae) puree and improves the fat separation from the pulp and from the protein fraction in the following separation step c) (Protein with less fat / lipids is obtained). Moreover, it has been established that the heated pulp obtained after step b) and c) of the method and the heated enzymatically hydrolysed pulp obtained after step a1) and step b) and step c) both have anti-oxidant properties.

A second aspect of the present invention relates to a fat fraction obtainable by the method according to the present invention. Since, the fat fraction of insects comprises a considerable amount of unsaturated fatty acids, reducing the heating time has a considerable effect on the quality of the fat fraction obtained, i.e. the fat fraction is less rancid than fractions obtained with methods already known in the prior art.

An aspect of the present invention relates to heated insect puree, preferably heated BSF larvae puree, obtained with the method of the invention. Such heated insect puree surprisingly has anti-oxidant properties and is suitable for use as an anti-oxidant feed or food or ingredient thereof.

An aspect of the present invention relates to hydrolysed insect puree, preferably hydrolysed BSF larvae puree. Hydrolysis of insect pure before the heating step, under influence of e.g. a peptidase, results in a puree with improved anti-oxidant activity. Moreover, protein separated from such hydrolysed puree comprises improvingly less fat compared to non-hydrolysed puree which is obtained according to the method of the invention, and compared to non-hydrolysed puree which is conventionally obtained by applying an extended heating step of e.g. 30 minutes.

An aspect of the present invention relates to heated insect puree, preferably heated BSF larvae puree, obtained with the method of the invention. Such heated insect puree surprisingly has anti-oxidant properties and is suitable for use as a health promoting feed or food or ingredient thereof.

An aspect of the present invention relates to hydrolysed insect puree, preferably hydrolysed BSF larvae puree. Hydrolysis of insect pure before the heating step, under influence of e.g. a peptidase, results in a puree with improved anti-oxidant properties. Moreover, protein separated from such hydrolysed puree comprises improvingly less fat compared to non-hydrolysed puree which is obtained according to the method of the invention, and compared to non-hydrolysed puree which is conventionally obtained by applying an extended heating step of e.g. 30 minutes.

A third aspect of the present invention relates to a protein fraction obtained with the method according to the present invention. Due to the lower/shorter temperature-time-combination the proteins have entrapped less fat than known insect protein fractions. Moreover, due to the relatively mild treatment conditions at least a part of the protein will have retained their functionality. Furthermore, the relatively mild treatment conditions reduces discolouration for example by Maillard reaction, compared to discolouration induced by larger treatment time and/or by treating at higher temperature for longer. This allows the use of various applications of said protein fraction.

An aspect of the present invention relates to a protein fraction obtained with the method according to the present invention, wherein the method contained the step of enzymatic hydrolysis of the insect pulp before the hydrolysed pulp was subjected to the step of heating the pulp. Due to the lower/shorter temperature-time-combination and the hydrolysis step, the proteins have entrapped less fat than known insect protein fractions. Furthermore, the relatively mild treatment conditions reduces discolouration for example by Maillard reaction, compared to discolouration induced by larger treatment time and/or by treating at higher temperature for longer. This allows the use of various applications of said protein fraction.

A fourth aspect of the present invention relates to the use of said fat fraction and/or protein fraction and/or insect puree and/or hydrolysed insect puree as a food or feed product, or as an ingredient thereof.

A fifth aspect of the present invention relates to insect pulp obtainable by steps a) and b), or steps a), a1) and b), of the method according to the present invention. Preferably, the insect pulp is black soldier fly larvae pulp.

A sixth aspect of the present invention relates to the use of the hydrolysed insect pulp and/or the insect pulp such as (hydrolysed) black soldier fly larvae pulp as an antioxidant food ingredient or feed ingredient, wherein the (hydrolysed) pulp is obtainable with the method of the invention, the method either or not involving the enzymatic hydrolysis step a1) prior to step b) of the method.

An aspect of the present invention relates to the use of the hydrolysed insect pulp and/or the insect pulp such as (hydrolysed) black soldier fly larvae pulp as a health-promoting food ingredient or feed ingredient, wherein the (hydrolysed) pulp is obtainable with the method of the invention, the method either or not involving the enzymatic hydrolysis step a1) prior to step b) of the method.

A seventh aspect of the invention relates to the use of the hydrolysed insect pulp and/or of the insect pulp such as black soldier fly larvae pulp as a food ingredient or as a feed ingredient, wherein the (hydrolysed) pulp is obtainable with the method of the invention, the method either or not involving the enzymatic hydrolysis step a1) prior to step b) of the method.

An aspect of the invention relates to the insect pulp or the hydrolysed insect pulp or derivative thereof according to the invention for use as feed or feed ingredient, in particular pet food.

An aspect of the invention relates to the use of the insect pulp or the hydrolysed insect pulp or derivative thereof according to the invention as a food or feed ingredient with health promoting potential.

An aspect of the invention relates to the insect pulp or the hydrolysed insect pulp or derivative thereof according to the invention for use in a method for the prevention and/or suppression of cellular oxidative damage.

### DEFINITIONS

The term *"insects"* as used herein refers to insects in any development stage, such as adult insects, insect larvae, insect prepupae and pupae.

The term *"hatching"* as used herein has its conventional meaning and refers to the process of young larvae emerging from the egg.

The term *"larvae"as* used herein has its conventional meaning and refers to the juvenile stadium of holometabolous insects, such as black soldier flies larvae.

The term *"hatchling"* or *"neonate"* as used herein has its conventional meaning and refers to larvae that have just hatched from the eggs.

The term *"prepupae"* as used herein refers to the last larval stage wherein the chitin content of the larvae has increased significantly.

The term *"pupae"* as used herein has its conventional meaning and refers to the stage of the insects life wherein the metamorphosis from larva to adult insect, such as black soldier flies

The term *"pulp"* or *"insect pulp"* or *"puree"* or *"larvae puree"* as used herein all have the same meaning and refers to the product obtained after mechanical size reduction of the insects to a size of less than 0.5 mm.

The term *"nutrient stream"* as used herein has its conventional meaning and refers to streams that contain nutrients, such as fats, protein and protein-derived material, carbohydrates, minerals and/or chitin. Within the context of the present invention, chitin is also considered a nutrient.

The term *"anti-oxidant property"* has its regular scientific meaning and here refers to a compound or a composition, such as the puree and the hydrolysed puree of the invention and obtainable with the method of the invention, that consists of or comprises an antioxidant with antioxidant activity, such as an anti-inflammatory response. Typically, such an anti-inflammatory response is a response to the inflammatory response induced by for example reactive oxygen species, e.g. in the cells of a mammal such as a pet or a human subject, or in the cells of a fish. Reference is made to for example book chapters 1, 5 and 6 of "Antioxidants in Food: Practical Applications" (Jan Pokorny, Nedyalka Yanishlieva and Michael Gordon (editors), 2001, Cambridge: CRC Press, Woodhead Publishing Ltd. ISBN 1 85573 463 X, CRC Press, ISBN 0-8493-1221-1). An anti-oxidant is a compound with anti-oxidant activity or a composition with anti-oxidant activity or a composition comprising a compound with anti-oxidant activity, such as activity against the oxidative damage resulting from host immune response. An anti-oxidant for example inhibits oxidation. Oxidation, *e.g.* reactive oxygen species, in a subject for example induces cellular (oxidative) damage.

The term *"health promoting",* such as in 'health promoting food', 'health promoting activity', 'health promoting property', and 'health promoting potential', has its regular scientific meaning and here refers to the effect of a compound or a composition, such as the hydrolysed puree or the puree of the invention or obtainable with the method of the invention, on the health of an animal such as a mammal such as a pet animal or a human subject, when such compound or composition is consumed by the animal. Consumption of the compound or composition with health promoting potential contributes to or supports or promotes or increases or maintains the health status of the animal such as a mammal such as a pet animal or a human subject. Reference is made to for example book chapters 1, 5 and 6 of "Antioxidants in Food: Practical Applications" (Jan Pokorny, Nedyalka Yanishlieva and Michael Gordon (editors), 2001, Cambridge: CRC Press, Woodhead Publishing Ltd. ISBN 1 85573 463 X, CRC Press, ISBN 0-8493-1221-1).

### BRIEF DESCRIPTION OF THE DRAWINGS

Figure 1 displays the fat separation from BSF larvae pulp as a function of heating time of the pulp.
Figure 2 depicts the influence of heating time of BSF larvae puree on colour of the insect pulp.
Figure 3A: Free amino-acid content in BSF larvae puree and in BSF larvae puree that was subjected to 0.1 wt% or 0.5 wt% peptidase based on the total weight of the puree.
Figure 3B: Pepsin digestibility of BSF larvae puree and of BSF larvae puree that was subjected to 0.1 wt% or 0.5 wt% peptidase based on the total weight of the puree.
Figure 3C: Content of lipids (fat) in protein meal isolated from BSF larvae puree and from BSF larvae puree that was subjected to 0.1 wt% or 0.5 wt% peptidase based on the total weight of the puree.
Figure 4: DPPH radical scavenging activity of BSF PureeX^{™} (BSF-P), BSF hydrolyzed puree (BSF-HP), Chicken meal (CM) and Fish meal (FM) after 30 min incubation (n=3).
Figure 5: DPPH radical scavenging activity of BSF PureeX^{™} (BSF-P), BSF hydrolyzed puree (BSF-HP), Chicken meal (CM) and Fish meal (FM) after 60 min incubation (n=3).
Figure 6: ABTS cation radical scavenging activity of BSF PureeX^{™} (BSF-P), BSF hydrolyzed puree (BSF-HP), Chiken meal (CM) and Fish meal (FM) after 30 min incubation (n=3).
Figure 7: MPO response modulation activity of BSF PureeX^{™} (BSF-P), BSF hydrolyzed puree (BSF-HP), Chicken meal (CM) and Fish meal (FM) using SIEFED assay (n=3).
Figure 8: MPO response modulation activity of BSF PureeX^{™} (BSF-P), BSF hydrolyzed puree (BSF-HP), Chicken meal (CM) and Fish meal (FM) using classical measurement (n=3).
Figure 9: Neutrophil response modulation activity of BSF PureeXTM (BSF-P), BSF hydrolyzed puree (BSF-HP), Chicken meal (CM) and Fish meal (FM) (n=3).
Figure 10: ABTS radical scavenging activity of WSEP performed in methanol. Results are mean ± SD of three independent assays in triplicate.

### DETAILED DESCRIPTION OF THE INVENTION

A first aspect of the present invention relates to a method to convert insects into nutrient streams, comprising the steps of:
a) providing fresh insects and preparing a pulp thereof;
b) heating the pulp for 50-100 seconds at a temperature of 60-95°C;
c) subjecting the heated pulp to a physical separation step thereby obtaining a fat fraction, an aqueous protein fraction and a solid-containing fraction. Throughout the description, examples, embodiments, claims, the terms "puree" and "pulp" have the same meaning.

It has surprisingly been found that with the method of the present invention it is possible to increase the yield of fat from the processed insects. It has further been found that by using the present time-temperature combination less oil is entrapped with the protein fraction. It has also been found that discolouration of the pulp and the nutrient fractions is reduced. The method of the present invention also saves energy as considerably less time is needed to heat the pulp, in contrast to time needed for methods previously suggested in the prior art.

An aspect of the present invention relates to a method to convert insects into nutrient streams, comprising the steps of:
a) providing fresh insects and preparing a pulp thereof; and
b) heating the pulp for 50-100 seconds at a temperature of 60-95°C, thereby obtaining an insect puree (pulp). The nutrient streams is an insect puree obtained after the heating of the prepared pulp from fresh insects, such as Black Soldier Fly (BSF, black soldier fly) larvae. Optionally, the method comprises a further step c) after step b): subjecting the heated pulp to a physical separation step thereby obtaining a fat fraction, an aqueous protein fraction and a solid-containing fraction. Drying of the protein fraction reveals protein meal obtained from insect such as BSF larvae.

An aspect of the present invention relates to a method to convert insects into nutrient streams, comprising the steps of:
a) providing fresh insects and preparing a pulp thereof;
a1) subjecting the pulp of step a) to enzymatic hydrolysis by contacting the pulp with a peptidase at a predetermined temperature and for a predetermined duration of time; subsequently b) heating the hydrolysed pulp of step a1) for 50-100 seconds at a temperature of 60-95°C, thereby obtaining a hydrolysed insect puree. The nutrient streams is a hydrolysed insect puree obtained after the heating of the hydrolysed pulp, the pulp obtained from fresh insects, such as BSF larvae. Optionally, the method comprises a further step c) after step b): subjecting the heated and hydrolysed pulp to a physical separation step thereby obtaining a fat fraction, an aqueous protein fraction and a solid-containing fraction. Drying of the protein fraction reveals hydrolysed protein meal obtained from insect such as BSF larvae.

The enzyme-treated insect pulp such as minced larvae, and (control) insect pulp without enzyme treatment (*e.g.* minced larvae that were not enzymatically hydrolysed), are for example heated to 90°C and the pulp is for example kept at this temperature for 80 seconds. The obtained insect puree can be subjected to tests for measuring free amino acid content and digestibility. Protein meal can be obtained from the heated insect pulp, such as heated BSF larvae pulp, and from the heated pulp after enzyme treatment before the heating step. For example, protein meal is obtained when batches of heated BSF larvae pulp that was first subjected to enzymatic hydrolysis of proteins using either 0.1 wt%, or 0.5 wt% Flavourzyme prior to the heating step at 90°C, *e.g.* by steps of protein separation from the pulp, evaporation, drying and grinding. It is part of the invention that fat is separated from the protein fraction to a higher extent when prior to heating the insect puree (BSF larvae puree) is enzymatically treated, compared to insect puree that is not enzymatically treated prior to heating. According to the invention, a dose dependent extent of fat separation from protein is obtained, when the amount of applied enzyme is considered. Thus, according to the invention, enzymatic treatment of *e.g.* minced larvae such as minced BSF larvae, using Flavourzyme prior to the heating step increases the free amino acid content and increases pepsin digestibility of the obtained hydrolysed larvae puree, and as a result, the obtained puree comprising hydrolysed protein has a better taste (free amino-acid content relates to attractive, appealable taste when animals and humans consume a product comprising free amino-acids), is highly digestible and has anti-oxidant properties (see test results, here below), according to the invention. In addition, enzymatic treatment of BSF larvae pulp improves the fat separation in the following separation step after enzymatic hydrolysis and heat-treatment of the enzymatically digested pulp, which increases the fat extraction from the protein meal, when compared to fat separation from the protein fraction obtained with larvae puree that was not subjected to an enzymatic hydrolysis step prior to heating at 90°C, according to the invention.

Although in the prior art methods are described for separating nutrient streams from insect pulp it has thus far not been recognized that a relatively mild treatment, *i.e.* relatively low and short temperature/time treatments could result in an increase yield of fat. In this regard reference is made to WO2014/123420 wherein a method for separating nutrient streams is described, but wherein it was considered that in order to increase the yield of fat obtained from the insect pulp relatively long and high temperature/time combinations should be used.

However, using long and high temperature/time combinations has a negative effect on the quality of the nutrient streams, in particular the fat fraction obtained. For example, if the insect pulp is heated for a relatively long time at a relatively high temperature a discolouration of the pulp occurs. Furthermore, since the fat fraction of the processed insects contains a relatively high amount of unsaturated fatty acids, the fat becomes rancid. This leads to off-flavours of the fat fraction and limits its scope of use.

With the method of the present invention these problems are largely overcome. First of all, with the method of the present invention discolouration of the insect pulp is greatly reduced. A further surprising advantage of the present method is that the fat fraction obtained from the insect pulp is less rancid than fat fractions obtained with methods known in the art. Moreover, the yield of fat from the processed insects is also considerably higher. Without wishing to be bound by any theory it is assumed that when using known methods a considerable part of fat is entrapped within denaturated proteins. By using milder treatment conditions this entrapment of fat can be avoided, resulting in a higher yield of fat from the insect pulp. Moreover, Maillard reactions with the proteins is occurring to a lesser extent due to the short heating time at mild temperature.

An embodiment is the method according to the invention, wherein the insects are insect larvae, preferably black soldier fly larvae.

An embodiment is the method according to the invention, wherein the black soldier fly larvae are between 12 and 30 days of age, preferably between 14 and 28 days, more preferably 14-26 days, most preferably 12 hours-3 days before the larvae transform into prepupae, such as 1-2 days before transformation.

An embodiment is the method according to the invention, wherein the pulp is not enzymatically treated prior to heating.

An embodiment is the method according to the invention, wherein the method further comprises a step a1) of treating the pulp by an enzyme prior to the step b).

An embodiment is the method according to the invention, wherein the method further comprises a step a1) of treating the pulp by an enzyme prior to the step b) and wherein the pulp is treated by the enzyme for 0,5 to 3 hours, preferably 1 to 2 hours at a temperature of 40°C to 70°C, preferably at 45°C to 65°C, more preferably at a temperature of 50°C ± 2°C.

An embodiment is the method according to the invention, wherein the method further comprises a step a1) of treating the pulp by an enzyme prior to the step b) and wherein the enzyme is a protease such as a peptidase, preferably a mixture of at least one protease and at least one peptidase, such as Flavourzyme.

An embodiment is the method according to the invention, wherein in step a) a pulp is prepared by mincing the insects.

An embodiment is the method according to the invention, wherein the average particle size of the remains of the insects in the pulp of step a) range between 10 and 500 micron.

An embodiment is the method according to the invention, wherein in step b) the pulp is heated for 60-90 seconds at a temperature of 60°C-95°C, particularly for 75-85 seconds at a temperature of 90°C ± 2°C.

An embodiment is the method according to the invention, wherein the physical separation step comprises decanting and/or centrifugation.

An embodiment is the method according to the invention, wherein the aqueous protein fraction and the solid containing fraction are dried together after step (c), preferably by spray-drying, more preferably fluidized bed drying.

An embodiment is the method according to the invention, wherein the aqueous protein fraction and the solid-containing fraction are dried separately after step c), wherein the aqueous protein fraction is preferably dried by spray drying, fluidized bed drying, lyophilisation or refractive drying, or a combination thereof.

As a result of the separation step, i.e. step c), a fat fraction, an aqueous protein fraction and a solid containing fraction are obtained. In this way, the method results directly in several nutrient streams. Although separation of the different fractions is usually the end-goal of the present invention, there are also application wherein it is advantageous to use the insect pulp obtained after step b). Hence, the present invention also relates to a method as described above, but only involving steps a) and b). Thereafter the pulp may be stored, packaged and possibly used as such as a food or feed ingredient. The present invention thus also relates to the insect pulp obtainable by or obtained by steps a) and b) of said method.

The insects used for preparing the insect pulp are preferably insect larvae, preferably larvae of holometabolous insects. The larvae are preferably made into an insect pulp before they become prepupae. Although it is possible to use prepupae for preparing insect pulp their chitin content is relatively high compared to 'normal' larvae before their transformation into prepupae, making them less suitable as a nutrient source for human or animal consumption. It is highly preferred to use larvae of the Black Soldier Fly, and most preferably the larvae have been cultivated but have not yet reached the prepupa stage. Typically they have been allowed to grow under normal growth conditions for 10-30 days, preferably 12-28 days, more preferably 14-24 days after hatching such as 14, 16, 18, 20, 22 days. For the aim of maximum yield of nutrient streams from the larvae, it is preferred that the larvae are subjected to the method of the invention shortly before the transformation of the larvae into the prepupae phase.

Therefore, it is advantageous to subject larvae to the method of the invention 12 hours to 3 days before the larvae start to transform into prepupae, preferably 1 day to 2 days before transformation into prepupae. Larvae such as Black soldier fly larvae, are at their largest size, containing maximum amounts of protein and fat compared to earlier and later stages in the larvae life cycle, when shortly before the transformation phase into prepupae. Moreover, larvae that start to transform intro prepupae discolour from light-yellow into gray/brown when larvae of Black soldier fly are considered, which is not desired when lightly coloured white/yellow protein is desired. Insect pulp and protein derived therefrom which has a darker, brownish/grayish colour due to application of prepupae in the method of the invention, is perceived as a product with inferior quality when compared to a lighter coloured product with a white/yellow appearance. In this regard it is noted that the options and preferences mentioned below are preferably carried out with insect pulp derived from such Black Soldier Fly larvae.

The preparation of a pulp from the insects - as referred to in step a) of the method of the present invention - may be done in different ways. One option is to squash the insects to obtain a pulp. Preferably, mincing, cutting or milling is used for preparing the pulp. This results in a homogeneous starting material of viscous consistency. The reducing in size can conveniently be done in a micro-cutter mill, although other suitable techniques may also be used. During this step, the particle size of the insects, preferably larvae, remaining in the pulp is preferably less than 500 micron, preferably between 10 and 500 micron (largest size determined by using a microscope). The particle size can be controlled by the selection of specific knife and plate combination and rotating speed in case of a micro-cutter mill. It is also possible to use a sieve (*e.g*. made of stainless steel) and push the insects through it. In principle a small remaining particle size is advantageous as it facilitates the fat extraction from the pulp, however a too small particle size could create an emulsion making it more difficult to separate the fat and protein in the next process steps. Hence, the particle size is preferably larger than 10 micron. Preferably, the average particle size (d₃₂; volume/average) ranges between 10 and 500 micron.

In the following step, step (b), the pulp is heated to a temperature of 60°C-95°C. The heating assures that the majority of fats is liquefied in order to prepare a suitable mixture for the following separation step. Preferably the heating is effectuated under mixing conditions to promote separation of different phases, such as the watery phase and fat phase. In order to avoid discolouration (e.g. by formation of Maillard reaction products) and oxidation of the fat and to improve fat yield the pulp is only heated at said temperature for a relatively short time, *i.e.* for 50 to 100 seconds. Preferably the pulp is heated to 60°C-95°C for 60-90 seconds, particularly for 75-85 seconds at a temperature of 90°C ± 2°C.

After heating the pulp to the above mentioned temperature, the pulp is subjected to a physical separation step, *i.e*. step (c) of the method of the present invention. During this step the various nutrient streams (such as fat and protein) are at least partly separated from each other. However, as explained above, the yield of fat is considerably higher with the method of the present invention as with methods known in the art. Furthermore, also the quality of the nutrient streams has improved. Preferably, the nutrient streams obtained are a fat containing fraction, an aqueous protein containing fraction and a solids containing fraction. The physical separation preferably encompasses decanting, centrifugation, or a combination of these methods. Preferably, the physical separation is performed at a normal (atmospheric) pressure.

The fat may be separated by means of decanting, and the remaining mixture is further separated into an aqueous protein fraction and a solid-containing fraction by decanting or centrifugation. However, it is also possible to obtain the fat, protein and solid-containing fractions in a different order, or simultaneously, *e.g*. by using a 3-phase decanter. An advantage of such a simultaneous method is that the three nutrient streams are obtained with a minimum of steps and thus with minimal losses of the product. Reducing the number of separation steps has also advantages when used in a continuous process.

In an embodiment of the present invention, a fat fraction is separated first, *e.g*. by decanting, and the remaining mixture is not further separated but subjected to drying. The remaining mixture therefore combines both the solid fraction and the aqueous protein fraction. In this embodiment, the non-fat phases are preferably further dried to produce dried material. The dried material is protein rich and contains both the protein-rich material from the aqueous protein fraction and solid-containing fraction. Since it is possible with the method of the present invention to better separate the fat from the protein a higher quality product is obtained. Furthermore, with the method of the present invention discolouration (of protein) due to Maillard reactions is largely avoided, thereby improving the quality of the product obtained. Drying can be effected by different methods, such as air drying, fluidized bed drying, drum drying, disc drying, flash drying, lyophilisation, refractive drying or preferably by means of spray drying.

In a further embodiment the aqueous protein fraction and the solid-containing fraction are dried separately after step c). Due to the relatively mild heating conditions at least a part of the proteins present in the aqueous protein fraction will have retained their functionality. It would be beneficial not to lose such functionality during subsequent drying. Hence, fluidized bed drying or spray drying or freeze drying or refractive drying is preferred in such circumstances.

A second aspect of the present invention relates to the fat fraction obtainable by the method according to the present invention. Since, the fat fraction of insects comprises a lot multiple unsaturated fatty acids, reducing the heating time has a considerable effect on the quality of the fat fraction obtained, *i.e*. the fat fraction is less rancid then fractions obtained with methods already known in the prior art. The fat fraction obtained by the method according to the present invention may be mixed with fat fractions obtained from other sources in order to obtain the desired properties. The present invention thus also relates to a fat composition comprising the fat fraction obtainable by the present invention.

An aspect of the present invention relates to heated insect puree, preferably heated BSF larvae puree, obtained with the method of the invention. Such heated insect puree surprisingly has anti-oxidant properties and is suitable for use as an anti-oxidant feed or food or ingredient thereof.

An aspect of the present invention relates to hydrolysed insect puree, preferably hydrolysed BSF larvae puree. Hydrolysis of insect pure before the heating step, under influence of *e.g*. a peptidase, results in a puree with improved anti-oxidant activity. Moreover, protein separated from such hydrolysed puree comprises improvingly less fat compared to non-hydrolysed puree which is obtained according to the method of the invention, and compared to non-hydrolysed puree which is conventionally obtained by applying an extended heating step of *e.g.* 30 minutes.

An aspect of the present invention relates to heated insect puree, preferably heated BSF larvae puree, obtained with the method of the invention. Such heated insect puree surprisingly has anti-oxidant properties and is suitable for use as a health promoting feed or food or ingredient thereof.

An aspect of the present invention relates to hydrolysed insect puree, preferably hydrolysed BSF larvae puree. Hydrolysis of insect pure before the heating step, under influence of *e.g.* a peptidase, results in a puree with improved anti-oxidant properties. Moreover, protein separated from such hydrolysed puree comprises improvingly less fat compared to non-hydrolysed puree which is obtained according to the method of the invention, and compared to non-hydrolysed puree which is conventionally obtained by applying an extended heating step of *e.g.* 30 minutes.

A third aspect of the present invention relates to a protein fraction obtained with the method according to the present invention. Due to the lower temperature-time-combination the proteins have entrapped less fat than known insect protein fractions. Moreover, due to the relatively mild treatment conditions at least a part of the protein will have retained their functionality. This allows the use of various applications of said protein fraction, which were previously not possible. Furthermore, with the method of the present invention discolouration (of protein) due to Maillard reactions is largely avoided, thereby improving the quality of the product obtained. The protein fraction may be in the form of the aqueous protein fraction obtained in step c) or may be a dried form thereof, *i.e.* in the form of a dried protein fraction. However, if one does not wish to lose the functionality of the protein a relatively mild drying method such as spray drying or freeze drying or refractive drying should be used.

The solid-containing fraction comprises a pulp having e a dry matter content of 50% by weight or more. This pulp can easily be distinguished and separated from the aqueous protein fraction. The pulp contains solids such as chitin and derivatives thereof. The solid-containing fraction may also comprise residual fat or protein, however, to a lesser extent than in known methods. This solid-containing fraction may be further dried and chitin may also be isolated therefrom. The chitin obtained may be used as an ingredient in food or feed.

An aspect of the present invention relates to the use of the hydrolysed insect pulp and/or the insect pulp such as (hydrolysed) black soldier fly larvae pulp as a health-promoting food ingredient or feed ingredient, wherein the (hydrolysed) pulp is obtainable with the method of the invention, the method either or not involving the enzymatic hydrolysis step a1) prior to step b) of the method.

A seventh aspect of the invention relates to the use of the hydrolysed insect pulp and/or of the insect pulp such as black soldier fly larvae pulp as a food ingredient or as a feed ingredient, wherein the (hydrolysed) pulp is obtainable with the method of the invention, the method either or not involving the enzymatic hydrolysis step a1) prior to step b) of the method.

An aspect of the invention relates to the insect pulp or the hydrolysed insect pulp or derivative thereof according to the invention for use as feed or feed ingredient, in particular pet food.

An aspect of the invention relates to the use of the insect pulp or the hydrolysed insect pulp or derivative thereof according to the invention as a food or feed ingredient with health promoting potential.

An aspect of the invention relates to the insect pulp or the hydrolysed insect pulp or derivative thereof according to the invention for use in a method for the prevention and/or suppression of cellular oxidative damage.

The inventors surprisingly established and analyzed *in vitro* anti-oxidant activity of black soldier fly proteins and protein hydrolysates using radical scavenging models (DPPH and ABTS assays), enzymatic models involving myeloperoxidases response (classical and SIEFED assays), and a cellular model involving neutrophil response. Commercial fish meal and chicken meal were used as industrial benchmarks in comparative examples. Outcomes of the tests and comparisons made by the inventors reveal that fish meal and chicken meal offer little to no advantage in terms of suppressing the oxidative damage occurring as a result of neutrophil and myeloperoxidase response. Moreover, fish meal and chicken meal also exhibit pro-oxidant behavior in some of the test assays and models used. Results show that black soldier fly proteins (as part of (hydrolysed) larvae puree) are effective in protecting against the cellular damage resulting from host neutrophil and myeloperoxidase response. Therefore, black soldier fly derivatives (puree, hydrolysed puree) tested by the inventors show advantages over chicken meal and fish meal for inclusion in pet food/feed and aquaculture feed formulations, such as use as a feed ingredient.

The inventors established that BSF proteins hydrolysates have a significant share of proteins <1000 Da. This includes a mixture of short chain peptides and free amino acids. Some short chain peptides and free amino acids are known to possess anti-oxidant activity. These molecules can actively scavenge ROS and free radicals. Firmansyah and Abduh [3] evaluated the DPPH (2,2-diphenyl-1-picrylhydrazyl) scavenging activity of BSF protein hydrolysates. Zhu et al. [4] evaluated the DPPH, ABTS (2,2'-azino-bis(3-ethylbenzothiazoline-6-sulfonic acid), superoxide and hydroxyl radical scavenging activity of BSF protein hydrolysates. No studies have been realized till date, to the best of the knowledge of the inventors, that established the antioxidant activity of BSF protein hydrolysate using fundamental enzymatic and cellular models, showing the surprisingly high anti-oxidant activity and surprisingly high protection against oxidative stress and/or damage by the BSF larvae puree and the hydrolysed BSF larvae puree. Therefore, before the currently established invention, the antioxidant potential of BSF protein hydrolysates is poorly understood on a fundamental level and was not yet disclosed, nor anticipated, nor expected. According to the invention, detailed investigations on the *in vitro* antioxidant activity of BSF proteins and protein hydrolysates unlock new applications of these protein derivatives to improve animal health.

The inventors unraveled the surprisingly high antioxidant potential of BSF proteins and protein hydrolysates, using: (1) radical scavenging models involving DPPH and ABTS; (2) enzymatic models involving myeloperoxidases response; and (2). a cellular model involving neutrophil response. Chicken meal and fish meal were used as industrial benchmarks in comparative examples for comparison with the anti-oxidant activities of the (hydrolysed) BSF larvae puree according to the invention.

The inventors surprisingly established that insect puree heated at 90°C for 40 s has relatively high amounts for free fatty acids (approx. 70%). See for example Example 4, relating to subjecting minced black soldier fly larvae to the method of the invention. Heating puree just for 40 s is ineffective in the deactivation of lipase. Whereas, low amounts of free fatty acids are observed when puree is heated for 80 s, indicating the sufficiency of this time for enzyme inactivation at 90°C. At heating times of over 80 seconds, such as up to 300 seconds or 1800 seconds, the fatty acid content increases when compared to the low fatty acid content obtained when the black soldier fly larvae puree is heated for 80 seconds. Therefore, for obtaining a protein fraction which comprises a relatively low free fatty acid content with the method of the invention, the heating time in the method of the invention should be longer than 40 seconds and shorter than 300 seconds, such as 50 - 100 seconds, or a heating time selected from 60 - 90 seconds, or 70 - 85 seconds, such as about 80 seconds. Preferred heating time is about 90°C or 90°C ± 2°C.

Surprisingly, the inventors established that higher peroxide value is observed for heating duration of less than 50 seconds, such as about 40 s, when minced insect pulp such as puree obtained with mincing black soldier fly larvae, is subjected to the method steps of the invention, though with a heating step duration of less than 50 seconds (therewith deviating from the method of the invention). The high amounts of free fatty acids that could participate in enzyme oxidation leading to the production of hydroperoxides, obtained when the puree or pulp is heated for 40 seconds, is at the basis for the occurrence of the relatively high amount of peroxides. However, when insect pulp or puree such as puree obtained from black soldier fly larvae, is subjected to the method of the invention, with a heating step of 80 s treatment, a relatively lower peroxide value is obtained, compared with the value obtained when heating for shorter than 50 seconds. Heating the product (*e.g.* puree from larvae) for a longer period than 100 seconds or less according to the method of the invention, such has for 300 seconds or 1800 seconds, has an effect on fat oxidation in that the peroxide value increases compared for example to the peroxide value when the puree or pulp is heated for a time of 80 seconds. Therefore, for obtaining a protein fraction which comprises a relatively low peroxide value using the method of the invention, the heating time in the method of the invention should be longer than 40 seconds and shorter than 300 seconds, such as 50 - 100 seconds, or a heating time selected from 60 - 90 seconds, or 70 - 85 seconds, such as about 80 seconds. Preferred heating time is about 90°C or 90°C ± 2°C.

Surprisingly, the inventors established that the protein fraction obtained with or provided by the method of the invention has radical scavenging activity. The radical scavenging activity is higher than the radical scavenging activity obtainable with conventional fish feed fed to fish, when the oxidation state of fish flesh is assessed after feeding the fish feed comprising a series of doses of black soldier fly larvae protein obtained with the method of the invention (lowest dose: 0% BSF larvae protein in the feed; highest dose: 100% BSF larvae protein; 25% and 50% are also tested, based on the total mass of the fish feed fed to fish). See also the examples section, here below, Example 5. ABTS radical scavenging activity of fish flesh after the fish were fed previously with feed comprising or consisting of protein obtained with the method of the invention (referred to as Water soluble extract powders (WSEP)) was compared with radical scavenging activity of fish flesh after the fish were fed previously with conventional feed lacking the black soldier fly larvae protein fraction obtained with the method of the invention. For all the samples increase in concentration resulted in an increase of mean inhibition values (showing the anti-oxidant behaviour of the fed feed when radical scavenging activity in the fish flesh is assessed). Eₘₐₓ (maximum inhibition obtained during the test) were in the following order: HI-25 > HI-100 > HI-50 > HI-0 (all obtained at 0.2 mg/ml, final concentration), referring to fish feed comprising 25, 100, 50 and 0 weight % BSF larvae protein obtained with the method of the invention, respectively, based on the total mass of the fed feed. This result show that at the dose of the HI-25 sample, the highest ABTS radical scavenging activity is obtained. On the other hand, HI-0 samples exhibit least ABTS radical scavenging activity. As assessed by using the ABTS assay, all the protein samples obtained by applying the method of the invention with black soldier fly larvae, exhibit anti-oxidant activity in a dose-dependent manner. HI-25 samples show the highest anti-oxidant activity.

In summary, with the method of the present invention it has become possible to obtain valuable nutrient streams from insects. These streams are not contaminated by foreign chemicals and have a purely biologic origin. The isolated nutrient streams may be used in the preparation of food, feed or pharmaceuticals. Moreover, depending on the intended application the nutrient streams obtained may be processed further, *e.g.* to isolate specific ingredients, such as hydrolysed protein, amino acids or specific fatty acids.

An embodiment is the method to convert insects into nutrient streams according to the invention, wherein in step b) the pulp is at a temperature of 75°C-95°C, particularly at a temperature of 80°C-93°C, preferably 85°C-90°C. An embodiment is the method to convert insects into nutrient streams according to the invention, wherein in step b) the pulp is heated for 60-95 seconds, particularly for 70-90 seconds, preferably 75-85 seconds such as 78 - 82 seconds. As said, the inventors established that by applying the (black soldier fly) larvae processing method of the invention, in step a) of the method, a nutrient stream comprising a stream of aqueous protein fraction obtained from black soldier fly larvae as the protein source, is obtained which aqueous protein fraction has surprisingly improved features and structural characteristics, when compared to protein obtained from such larvae by applying an alternative black soldier fly larvae processing method known in the art, such as the method outlined in European patent application EP2953487, in the Examples section thereof, in particular Example 1, page 12, line 8-13 and page 13, line 3-5. In particular, the relatively short heating time of 100 seconds or less than 100 seconds, in step b) of the method of the invention, is deemed essential for obtaining improved larvae pulp in step b) and subsequently for obtaining improved aqueous protein fraction in step c) of the method. It has been found by the inventors that by using the time-temperature combination less oil is entrapped within the aqueous protein fraction, therewith providing a more pure protein source in step c) of the method of the invention, compared with protein sources provided using alternative, current means and methods for isolating a protein fraction from black soldier fly larvae as the protein source. It has also been found that discolouration of the heated pulp (of protein) in step b) of the method, due to Maillard reactions, is largely avoided, thereby improving the quality of the product obtained, *e.g.* the heated pulp in step b) and the aqueous protein fraction in step c) of the method of the invention. For example, aqueous protein fraction with reduced fat content is obtained when minced black soldier fly larvae are heated for 80 seconds at 90°C, or for 75-85 seconds at a temperature of 90°C ± 2°C. In EP2953487, heating time is at least 5 minutes and even 30 minutes. Furthermore, the relatively short heating time of at most 100 seconds, preferably about 70-90 seconds, of the minced black soldier fly larvae pulp (provided in step a) of the method) results in less granulated and less aggregated heated insect pulp in step b) of the method compared to longer heating times applied in current methods known in the art. Less granulated larvae pulp after step b) of the method is beneficial for, for example, the enzymatic hydrolysis of the protein comprised by the pulp, and in general, a less granulated and aggregated insect pulp and subsequent protein fraction contribute to improved separation results and improved ease of processing the pulp and protein. For example, granulation reduces enzyme efficiency since the particulate matter is less accessible for the enzyme molecules. By applying the larvae processing method steps as part of the method of the invention, the short heating time results in a protein composition consisting of smaller protein clusters, aggregates, particles, or even (mainly) of protein monomers and/or small multimers, compared with the larger protein aggregates and particles obtained when black soldier fly larvae pulp is subjected to heating for over 100 seconds such as for 5 minutes up to 30 minutes. In addition, fat is entrapped inside the relatively large protein pulp clusters and protein fraction aggregates after heating the insect pulp for longer times than 100 seconds, for example at 90°C.

The present invention will be illustrated further by means of the following non-limiting examples.

### EXAMPLES & EMBODIMENTS

### Example 1

Live and washed larvae of 14 days old (5 kg) were collected just before the mincer and stored at 4°C until used. For each experiment 100 g larvae were minced freshly. 100 g of minced larvae were heated to 90°C (using a hot plate and with continuously stirring) and the product was kept at this temperature for 0, 40, 80, 120, 300, 600 and 1800 s. Approximate 30 g of heated larvae were collected in 3 centrifugable tubes and centrifuged at 3200G for 5 minutes. The fat fraction was extracted after centrifugation and were collected using a Pasteur pipette and weighed to calculate the % fat separation (of total puree weight on wet basis).

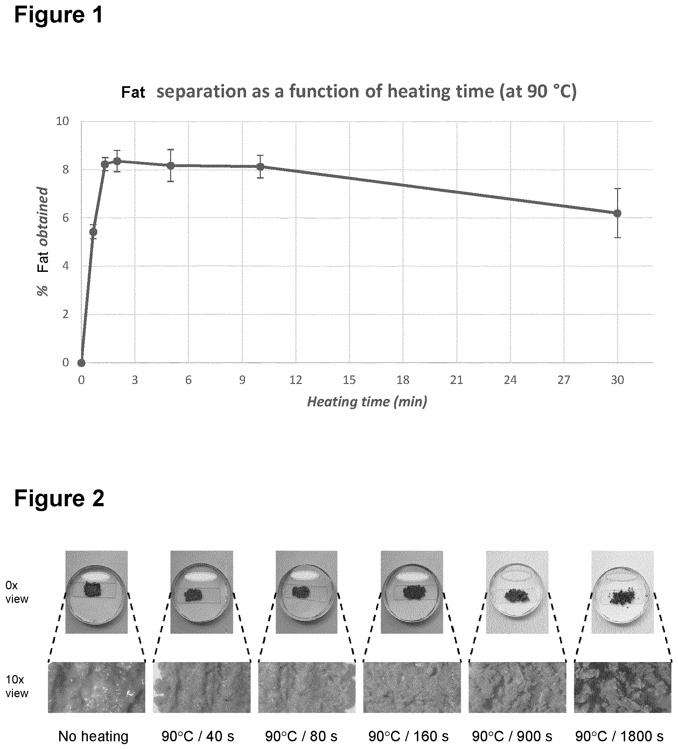

The fat separation as a function of time is provided in table 1 below and Figure 1. From this it is clear that upon centrifugation of unheated insect pulp no fat was obtained, but that it steadily increased till a heating time of 80 seconds. Thereafter it remained stable until the heating time of 600 seconds, after which the fat separation started to decline. The surprising results show that different from what was expected in the art, relatively short heating times of 10 minutes or less may be used for the most efficient removal of fat from the heated insect pulp. Furthermore, due to the reduced heating times less discolouration of the heated insect pulp occurred, which leads to a product (and derived nutrient streams) with a higher quality. In Figure 2 the influence of heating time on colour of the insect pulp is depicted. Moreover, the short heating time results in less granulated and less aggregated insect pulp compared to longer heating times of over 600 seconds (see *e.g.* Figure 2).

**Table 1: fat separation as a function of heating time (at 90°C)**

| Heating time (s) | % fat obtained |
|---|---|
| 0 | 0 |
| 40 | 5.7 |
| 80 | 8.2 |
| 120 | 8.5 |
| 300 | 8.4 |
| 600 | 8.1 |
| 1800 | 6.2 |

### Example 2

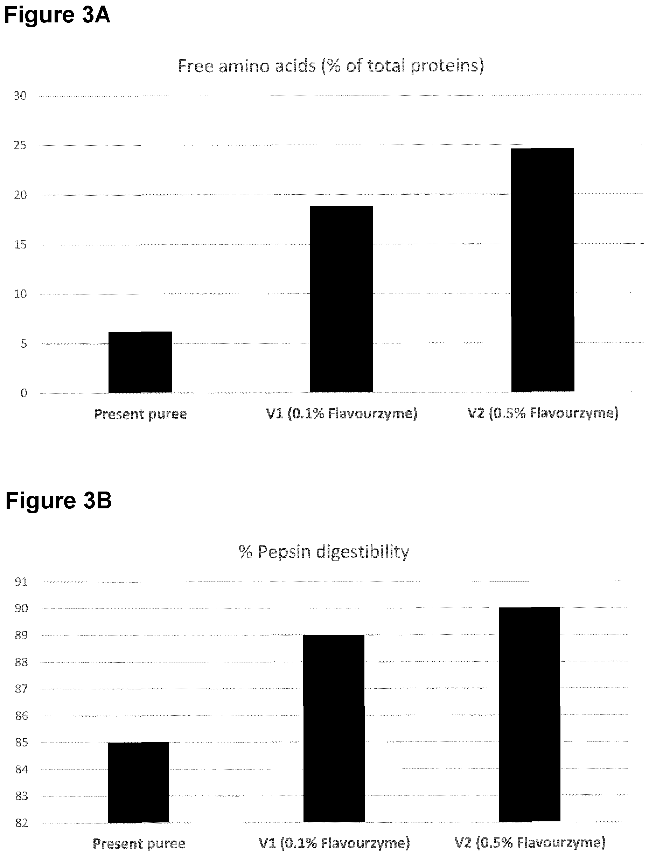

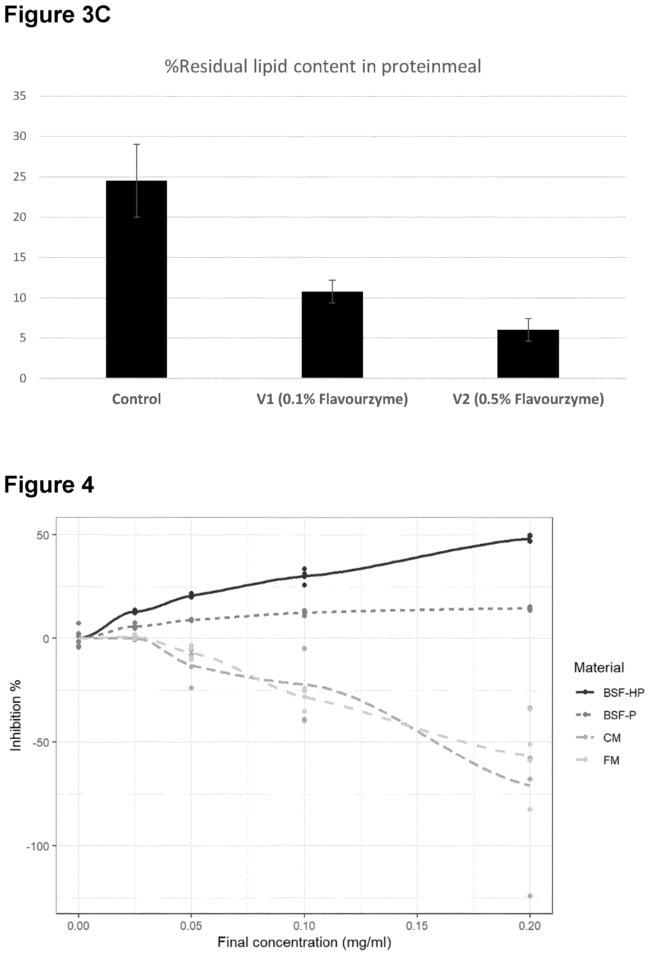

A puree of BSF larvae, also referred to as BSF PureeX^{™} (BSF-P), and hydrolyzed puree (BSF-HP) obtained by enzymatic hydrolysis of the puree of BSF larvae, were prepared by Protix B.V. (Dongen, The Netherlands) in October 2019. The puree and the enzymatically digested puree were obtained according to the following method. Live and washed Black Soldier Fly larvae of 14 days old were collected just before being subjected to the mincer for mincing the larvae (therewith providing larvae pulp), and subsequently stored at 4°C until used. For each experiment, larvae were minced freshly. The minced larvae was treated with 0.1% or 0.5% Favourzyme, based on the mass of the minced larvae, for 0,5 - 3 hours, for example 1 to 2 hours, at 45°C to 65°C (± 2°C) under continuous stirring. The batch of hydrolysed BSF larvae puree applied in Example 2 was obtained by enzymatic hydrolysis for 1 hr at 50°C (± 2°C). Flavourzyme (Novozymes, Denmark) is a combination of aminopeptidases which have endopeptidase and exopeptidase activities. The enzyme-treated minced larvae, and control larvae without enzyme treatment, were heated to 90°C and the product was kept at this temperature for 80 seconds. The obtained insect puree were used for measuring free amino acid content (Figure 3A: Free amino acid content (n=1)- Tested in puree) and pepsin digestibility (Figure 3B: Digestibility (n=1)- Tested in puree). Protein meal was obtained from the heated BSF larvae pulp, and from the two batches of heated BSF larvae pulp that was first subjected to enzymatic hydrolysis of proteins using either 0.1 wt%, or 0.5 wt% Flavourzyme prior to the heating step at 90°C, e.g. by steps of protein separation from the pulp, evaporation, drying and grinding. The percentage of fat separation was measured during the separation step (Figure 3C: % fat separation (n=3)- Tested during separation step to make protein meal). In all experiments performed, a dose dependent effect was observed, when the amount of applied enzyme is considered. Thus, enzymatic treatment of minced larvae using Flavourzyme prior to the heating step increases the free amino acid content and increases pepsin digestibility of the obtained hydrolysed larvae puree, and as a result, the obtained puree comprising hydrolysed protein has a better taste (free amino-acid content relates to attractive, appealable taste when animals and humans consume a product comprising free amino-acids), is highly digestible and has anti-oxidant properties (see test results, here below). In addition, enzymatic treatment of BSF larvae pulp improves the fat separation in the following separation step after enzymatic hydrolysis and heat-treatment of the enzymatically digested pulp, which increases the fat extraction from the protein meal, when compared to fat separation from the protein fraction obtained with larvae puree that was not subjected to an enzymatic hydrolysis step prior to heating at 90°C.

### Example 3

### 2. Materials and Methods

### 2.1. Reagents

All the reagents were of analytical grade. Dimethylsulfoxide, methanol, ethanol, calcium chloride, potassium chloride, sodium chloride, hydrogen peroxide and Tween-20 were purchased from Merck (VWR, Leuven, Belgium). Sodium nitrite, bovine serum albumin, phorbol 12-myristate 13-acetate and Percoll^{™} were purchased from Sigma (Bornem, Belgium). Aqueous extracts and solutions were made in Milli-Q water obtained using Milli-Q water system (Millipore, Bedford, USA). Bicinchoninic acid and copper (II) sulfate solutions were purchased from Sigma (Steinheim, Germany). Whatman filter paper grade 4 (270 mm) was purchased from Amersham (Buckinghamshire, UK). Sterlip 30 ml disposable vacuum filter system was purchased from Millipore (Bedford, USA). 2,2-Diphenyl-1-picrylhydrazyl and 2'-azino-bis(3-ethylbenzthiazoline-6-sulfonic acid) were purchased from Aldrich (Darmstadt, Germany). 8-amino-5-chloro-7-phenylpyrido[3,4-d]pyridazine-1,4(2H,3H)dione (L-012) was purchased from Wako Chemicals (Neuss, Germany).

### 2.2. Raw materials

Chicken meal (CM) and fish meal (FM) were purchased from an online webshop in September 2019. The chemical composition of both ingredients as declared by the supplier is indicated in table 2.

**Table 2. Chemical composition of chicken meal and fish meal (as in basis, provided by supplier).**

| **Nutrients** | **Chicken meal** | **Fish meal** |
|---|---|---|
| Moisture (g/kg) | 60.0 | 100.0 |
| Crude protein (g/kg) | 700.0 | 710.0 |
| Crude fat (g/kg) | 120.0 | 120.0 |
| Added antioxidant | No | Yes (E324) |
| Form | Powder | |

BSF-P and hydrolyzed puree BSF-HP were prepared by Protix B.V. (Dongen, The Netherlands) in October 2019. (1). BSF-P was pasteurized BSF minced 'meat' (puree, pulp) supplied frozen at -20°C. BSF-P is also the raw material to produce BSF protein meal (ProteinX^{™}). (2). BSF-HP was hydrolyzed and pasteurized BSF meat (puree) also supplied frozen at -20°C. (3). The chemical composition of the two ingredients BSF-P and BSF-HP were as is indicated in table 3.

**Table 3. Chemical composition of BSF protein derivatives heated puree and heated hydrolysed puree (as in basis, provided by supplier).**

| **Nutrients** | **BSF-P¹** | **BSF-HP²** |
|---|---|---|
| Moisture (g/kg) | 700.0^{a} | 700.0^{a} |
| Crude protein (g/kg) | 120^{a} | 120^{a} |
| Crude fat (g/kg) | 122.5^{a} | 122.5^{a} |
| Added antioxidant | No | No |
| % of total proteins <1000 Da | >6 | >24 |
| Form | Frozen minced meat | |

| | | |
|---|---|---|
| ¹BSF-P: BSF PureeX^{™}; ²BSF-HP: BSF hydrolyzed puree; ^{a}Mean values based on the range established at Protix. | | |

Water soluble extracts were prepared for CM, FM, BSF-P and BSF-HP. These products (100 g each) were dissolved with six times volumes of Milli-Q water based on their respective dry matter contents (*e.g.* BSF-P had dry matter content of 33.3% and was diluted 200 ml Milli-Q water) and stirred for 2 h on a magnetic stirrer. Post centrifugation (1000 x g for 30 min at 4°C), the top fat layer was removed and the supernatant was filtered using Whatman Filter (grade 4). The centrifugation and filtration step was repeated again to remove all non-soluble residues. Finally the supernatant was filtered using a Sterlip Filter (50 mL, 0.22 µm) and freeze dried over a period of two days to obtain respective water soluble extract powders. All four water soluble extract were stored in a desiccator (at 18°C) until further use.

### 2.3. Protein quantification

Total protein content of the four water soluble extracts was analysed using Bicinchoninic acid (BCA) protein assay *[5].* The calibration curve was obtained using bovine serum albumin (BSA) as standard at concentrations: 0, 0.125, 0.25, 0.5 and 1 mg/ml. Stock solutions of 3 mg/ml water soluble extracts were used for analysis. A test solution was made by dissolving 4900 µl BCA (49/50) and 100 µl copper (II) sulfate (1/50). Sample stock solutions (10 µl) and test solution (200 µl) were added in wells of 96-well plate. This plate was incubated at 37 °C for 30 min and absorbance was measured at 450 nm using a Multiscan Ascent (Fisher Scientific, Asse, Belgium).

### 2.4. DPPH assay

DPPH radical scavenging activity was analysed according to protocol of Brand-Willams et al. *[6],* with some modifications. DPPH test solution was made by dissolving 10.5 mg DPPH in 40 ml ethanol. Test solution was made fresh and stored in dark until further use. DPPH working solution was made by diluting test solution with 10 times ethanol (to obtain absorbance of 0.6 to 0.8 at 517 nm). DPPH working solution (1920 µl) was mixed with 20 µl of samples dilutions (four water soluble extracts in Milli-Q water) to obtain final concentration of 0.0125, 0.025, 0.05, 0.1 and 0.2 mg/ml. The decrease in absorbance after 30 and 60 min of incubation in dark was recorded at 510 nm using HP 8453 UV-vis spectrophotometer (Agilent Technologies, Waldbronn, Germany). Instead of sample dilutions only Milli-Q water was used in case of control.

### 2.5. ABTS assay

ABTS cation radical scavenging activity was analysed according to protocol of Arnao et al. *[7],* with some modifications. ABTS test solution was made by dissolving 7.0 mmol/l ABTS and 2.45 mmol/l potassium persulfate in Milli-Q water. The test solution was kept overnight in dark at room temperature. ABTS working solution was made by diluting with methanol to obtain the absorbance between 0.7 and 0.8 at 734 nm. ABTS working solution (1920 µl) was mixed with 20 µl of samples dilutions (four water soluble extracts in Milli-Q water) to obtain final concentration of 0.0125, 0.025, 0.05, 0.1 and 0.2 mg/ml. The decrease in absorbance after 30 min of incubation in dark was recorded at 734 nm using HP 8453 UV-vis spectrophotometer (Agilent Technologies, Waldbronn, Germany). Instead of sample dilutions only Milli-Q water was used in case of control.

### 2.6. Myeloperoxidase (MPO) activity using Specific Immunological Extraction Followed by Enzymatic Detection (SIEFED) assay

SIEFED assay is a licensed method developed by Franck et al. *[8]* for specific detection of animal origin MPO. MPO solution was made by diluting human MPO in 20 mM phosphate buffer saline (at pH 7.4), 5 g/l BSA and 0.1% Tween-20. Sample dilutions at final concentration of 0.0125, 0.025, 0.05, 0.1 and 0.2 mg/ml were incubated for 10 min (at 37 °C) with MPO solution at a final concentration of 25 ng/ml. After incubation, the mixtures were loaded into the wells of a 96 wells microtitre plate coated with rabbit polyclonal antibodies (3 µl/ml) against equine MPO and incubated for 2 h at 37 °C in darkness. After washing up the wells, the activity of the enzymes captured by the antibodies was measured by adding hydrogen peroxide (10 µM), NO₂ (10 mM) and Amplex^{™} Red (40 µM). The oxidation of Amplex^{™} Red into the fluorescent adduct resorufin was monitored for 30 min at 37 °C with Fluorosckan Ascent (Fisher Scientific, Asse, Belgium). Instead of sample dilutions only Milli-Q water was used in case of control.

### 2.7. Myeloperoxidase (MPO) activity using classical measurement

MPO solution was prepared as mention in section 2.6. Sample dilutions at final concentration of 0.0125, 0.025, 0.05, 0.1 and 0.2 mg/ml were incubated for 10 min (at 37 °C) with MPO solution at a final concentration of 25 ng/ml. After incubation, the mixture (100 µl) was immediately transferred into 96-well microtitre plate. This was followed by addition of 10 µl NO₂⁻ (10 mM) and 100 µl of Amplex^{™} Red and hydrogen peroxide mixture (at concentrations mentioned in section 2.6). The oxidation of Amplex^{™} Red into the fluorescent adduct resorufin was monitored for 30 min at 37 °C with Fluorosckan Ascent (Fisher Scientific, Asse, Belgium) immediately after addition of relevation mixture. Instead of sample dilutions only Milli-Q water was used in case of control.

### 2.8. Cellular antioxidant activity

Preparation of the neutrophil and phorbol 12-myristate 13-acetate (PMA) solutions were made according to Paul et al. *[2].* Neutrophil response modulation activity of samples was analysed using the protocol of Tsumbu et al. *[1].* Neutrophil suspension (1 million cells/143 µl PBS) was loaded in wells of 96-wells microtite plate and incubated for 10 min (at 37°C in dark) with phospahte buffer saline solution of samples at final concentrations of 0.0125, 0.025, 0.05, 0.1 and 0.2 mg/ml. After incubation, 25 µl calcium chloride (10 µM) and 20 µl L-012 (100 µM) were added in wells. The neutrophils were activated with 10 µl PMA (16 µM) immediately before monitoring the chemiluminesence response of neutrophils during 30 min at 37°C using Fluorosckan Ascent (Fisher Scientific, Asse, Belgium). Instead of sample dilutions only phosphate buffer saline was used in case of control.

### 2.9. Statistical analyses

All the analyses were performed in triplicates. For protein quantification, the equation of a fitted line and R-square value were calculated using linear regression. The relationships between concentration and inhibition obtained for antioxidant assays were non-monotonic in nature. To address this, the locally estimated scatterpot smoothing (LOESS) regression technique was used to model the relationship *[9].* Models were fitted using the R statistical software *[10].* These models require a span parameter that defines the smoothing sensitivity of the local regressions. By visual inspection a span parameter value of 0.4 was found to be suitable for all concentration and inhibition relationship curves. Concentrations with a predicted inhibition percentrage of interest, such as IC₅₀ (concentration at which 50 % inhibition is reached), were found using the fitted models in combination with a numerical search routine.

### 3. Results

### 3.1. Protein quantification

The calibration curve obtained using BSA, equation of the line and R-square value are established. The optical density of samples and relative concentration of proteins (calculated using equation of line) are mentioned in table 3. BSF-P extract solution (3 mg/ml) exhibits the highest, on the other hand BSF-HP solution exhibits the lowest protein concentrations amongst the tested solutions using Bichinchoninic acid assay.

**Table 4. Protein quantification using Bichinchoninic acid assay**

| **Product** | **Product used for testing in all the assays** | **Mean optical density** | **Protein concentration (mg/ml)** |
|---|---|---|---|
| BSF-P¹ | Water soluble extract | 0.486 | 1.013 |
| BSF-HP² | Water soluble extract | 0.365 | 0.648 |
| FM³ | Water soluble extract | 0.425 | 0.829 |
| CM⁴ | Water soluble extract | 0.481 | 0.998 |

| | | | |
|---|---|---|---|
| ¹BSF-P: BSF PureeX^{™}; ¹BSF-P: BSF hydrolyzed puree;: Fish meal; ⁴CM: Chicken meal. | | | |

### 3.2. DPPH assay

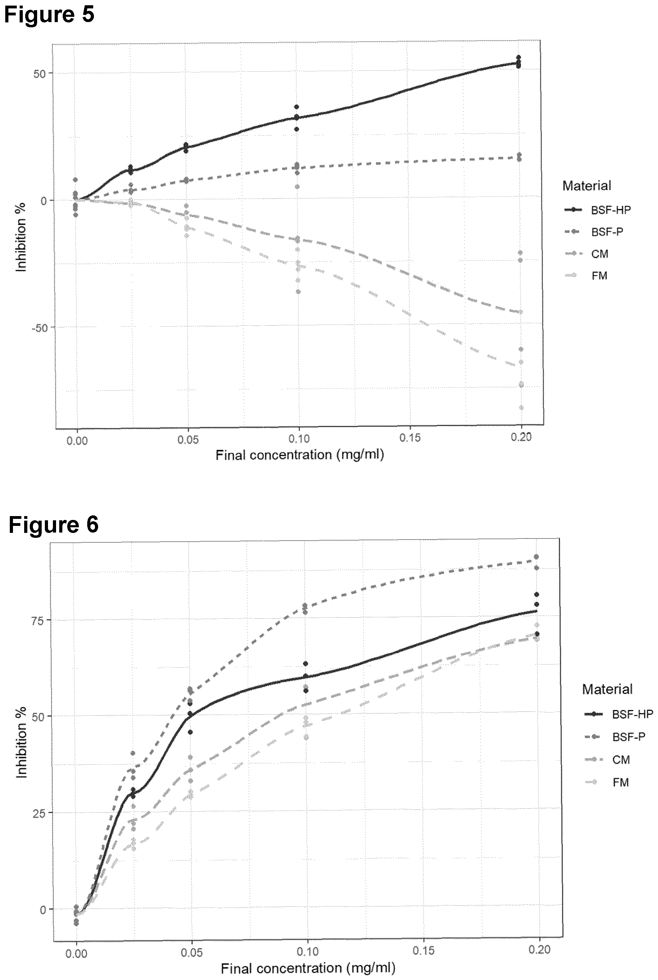

DPPH radical scavenging activity of all five samples after 30 and 60 minutes of incubation is indicated in figure 4 and figure 5, respectively. The plot shows the measured values as well as fitted curves obtained from LOESS. CM exhibited pro-oxidant behavior at all tested concentrations after 30 as well as 60 minutes of incubation. Whereas, FM exhibited pro-oxidant behavior at four out of five tested concentrations after 30 min of incubation and at all tested concentrations after 60 min of incubation. The IC₅₀ of BSF-HP after 60 min of incubation is indicated in table 5. It was not possible to calculate IC₅₀ for other samples (after 30 or 60 min of incubation) because the samples either exhibited pro-oxidant activity or 50% inhibition was not achieved during the assay. The Eₘₐₓ (maximum inhibition achieved during the assay) of all the samples are also indicated in table 6 and are in following order: BSF-HP> BSF-P> FM and BSF-HP> BSF-P after 30 and 60 minutes of incubation, respectively.

**Table 5. Antioxidant activity IC₅₀ (mg/ml) of samples obtained using different assays**

| **Assay** | **BSF-P¹** | **BSF-HP²** | **FM³** | **CM⁴** |
|---|---|---|---|---|
| **DPPH 30 min** | NE^{c} | NE^{c} | NE^{c} | PO^{d} |
| **DPPH 60 min** | NE^{c} | 0.18 | PO^{d} | PO^{d} |
| **ABTS 30 min** | 0.04 | 0.05 | 0.11 | 0.09 |
| **MPO^{a} SIEFED** | NE^{c} | 0.14 | PO^{d} | PO^{d} |
| **MPO^{a} Classical** | 0.10 | 0.09 | PO^{d} | PO^{d} |
| **CAA^{b}** | 0.15 | 0.15 | NE^{c} | NE^{c} |

| | | | | |
|---|---|---|---|---|
| ¹BSF-P: BSF PureeX^{™}; ²BSF-HP: BSF hydrolyzed puree; ³FM: Fish meal; ⁴CM: Chicken meal; ^{a}MPO: Myeloperoxidase; ^{b}CAA: Cellular antioxidant activity using neutrophil model; ^{c}NE: Not estimated because 50% inhibition was not achieved in tested concentrations; ^{d}PO: Not estimated because sample exhibited pro-oxidant activity on tested concentrations. | | | | |

**Table 6. Antioxidant activity Eₘₐₓ (% inhibition) of samples obtained using different assays**

| **Assay** | **Parameter** | **BSF-P¹** | **BSF-Hp2** | **FM³** | **CM⁴** |
|---|---|---|---|---|---|
| **DPPH 30 min** | **Emax (%)** | **14.52** | **48.09** | **0.75** | **PO^{c}** |
| | C* (mg/ml) | 0.20 | 0.20 | 0.03 | - |
| **DPPH 60 min** | **Emax (%)** | **15.22** | **52.53** | **PO^{c}** | **PO^{c}** |
| | C* (mg/ml) | 0.20 | 0.20 | - | - |
| **ABTS 30 min** | **Emax (%)** | **89.33** | **76.32** | **70.40** | **69.39** |
| | C* (mg/ml) | 0.20 | 0.20 | 0.20 | 0.20 |
| **MPO^{a} SIEFED** | **Emax (%)** | **36.23** | **77.58** | **PO^{c}** | **PO^{c}** |
| | C* (mg/ml) | 0.20 | 0.20 | - | - |
| **MPO^{a} Classical** | **Emax** (%) | **89.66** | **83.82** | **PO^{c}** | **PO^{c}** |
| | C* (mg/ml) | 0.20 | 0.20 | - | - |
| **CAA^{b}** | **Emax (%)** | **59.57** | **59.64** | **21.81** | **5.08** |
| | C* (mg/ml) | 0.20 | 0.20 | 0.05 | 0.20 |

| | | | | | |
|---|---|---|---|---|---|
| *C: Concentration at which Eₘₐₓ is achieved; ¹BSF-P: BSF PureeX^{™}; ²BSF-HP: BSF hydrolyzed puree; ³FM: Fish meal; ⁴CM: Chicken meal; ^{a}MPO: Myeloperoxidase; ^{b}CAA: Cellular antioxidant activity using neutrophil model; ^{c}PO: Not estimated because sample exhibited pro-oxidant activity on tested concentrations. | | | | | |

### 3.3. ABTS assay

ABTS cation radical scavenging activity of samples after 30 minutes of incubation is shown in figure 6 (measured values as well as fitted curves obtained from LOESS). All the samples exhibited a similar inhibition pattern *i.e.,* % inhibition increased as a function of increasing concentration. The IC₅₀ of samples are mentioned in table 5 and are in following order: FM> CM> BSF-HP > BSF-P. Lower the IC₅₀, higher is the ABTS cation radical scavenging activity. The Eₘₐₓ (maximum inhibition achieved during the assay) of all the samples are indicated in table 6 and are in following order: BSF-P> BSF-HP> FM> CM.

### 3.4. Myeloperoxidase (MPO) activity using Specific Immunological Extraction Followed by Enzymatic Detection (SIEFED) assay

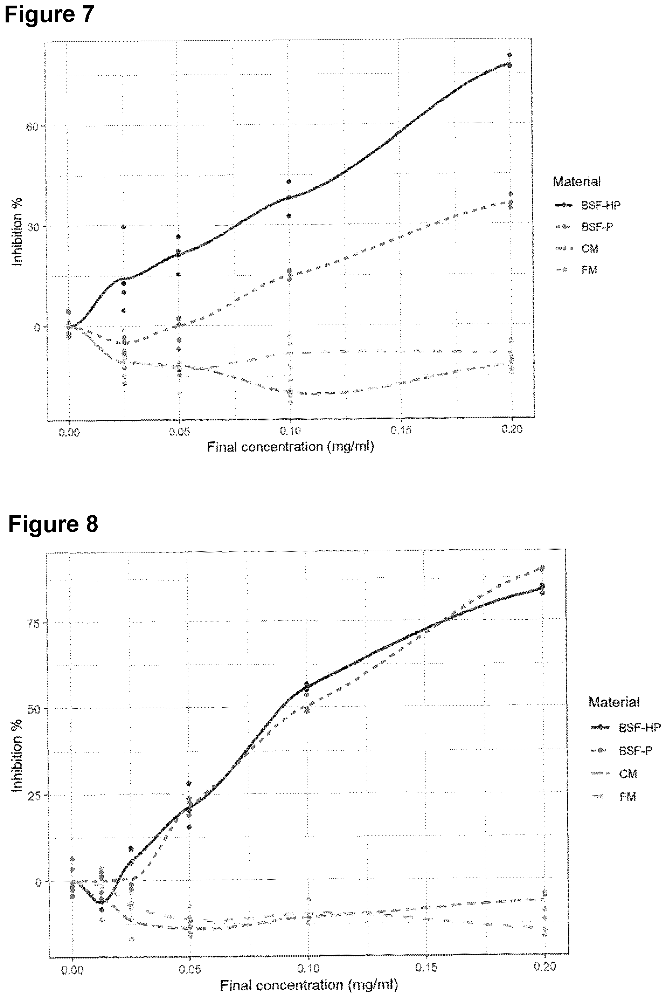

MPO response modulation activity of samples obtained using SIEFED assay is shown in figure 7 (measured values as well as fitted curves obtained from LOESS). BSF-HP exhibited strong inhibition behavior, with >75 % inhibition at 0.20 mg/ml concentration. The IC₅₀ of samples are mentioned in table 5 and are in following order: BSF-HP. The Eₘₐₓ of samples are shown in table 6, and are in following order: BSF-HP> BSF-P. FM and CM show pro-oxidant behavior at all tested concentrations. On the other hand Eₘₐₓ for BSF-P was < 50 %.

### 3.5. Myeloperoxidase (MPO) activity using classical assay

MPO response modulation activity of samples obtained using classical assay is indicated in figure 8 (measured values as well as fitted curves obtained from LOESS). CM and FM exhibited pro-oxidant behavior at all tested concentrations. The Eₘₐₓ of all the samples tested are indicated in table 6. BSF-P and BSF-HP exhibited Eₘₐₓ > 75 %. The IC₅₀ of samples are mentioned in table 5 and are in following order: BSF-P> BSF-HP.

### 3.5. Cellular antioxidant activity

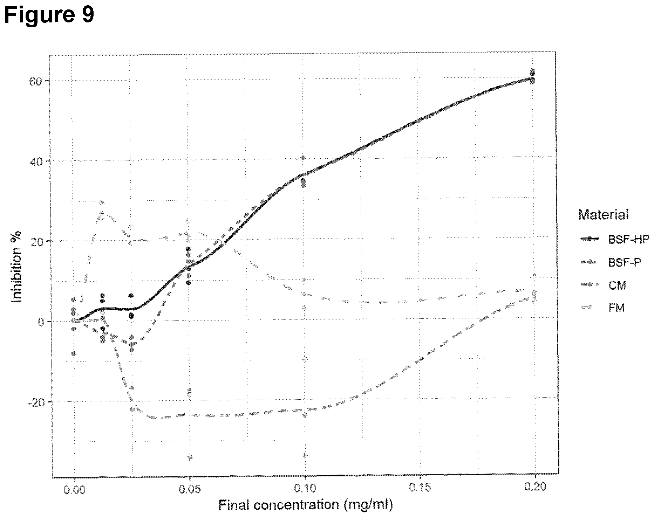

Neutrophil response modulation activity (measured values as well as fitted curves obtained from LOESS) and Eₘₐₓ of samples are shown in figure 9 and table 6, respectively. All the tested samples exhibited Eₘₐₓ > 0%. FM and CM exhibited Eₘₐₓ < 40%. CM exhibited pro-oxidant behavior at 3 out of 5 tested concentration. The IC₅₀ of samples are mentioned in table 5. BSF-P and BSF-HP have the same numerical IC₅₀ values.

### 4. Discussion

### 4.1. Protein quantification

The protein concentration of the four water soluble extracts estimated using Bichinchoninic acid assay are displayed in table 4. Considering the amino acid pattern similarities between black soldier fly proteins, FM and CM, the protein content of four water soluble extracts are in following order: BSF-P> CM> FM> 45.5 %> BSF-HP.

### 4.2. DPPH radical scavenging activity

DPPH and ABTS assays are commonly used to analyze antioxidant potential of food and feed products. DPPH radical scavenging activity represents the ability of a sample to donate hydrogen atom (referred as hydrogen atom transfer) or electrons (referred as single electron transfer) to stabilize free radicals. DPPH assay IC₅₀ and Eₘₐₓ for all tested samples are mentioned in table 5 and table 6, respectively. Post 30 min of incubation, all the tested samples exhibit Eₘₐₓ < 50 % (with BSF-HP exhibiting highest Eₘₐₓ). On the other hand, after 60 min of incubation only BSF-HP exhibit Eₘₐₓ > 50 %. BSF-HP is manufactured by controlled hydrolysis of black soldier fly proteins and contains at least 24 % of proteins <1000 Da (see table 3). On the other hand BSF-P contains at least 6 % proteins <1000 Da. The inventors were not able to find any representative literature for molecular weight distribution of FM and CM. However, according to the literature, FM and CM contain 2.2 % and 1.1 % free amino acid (of total proteins), respectively [Li, P.; Wu, G. Composition of amino acids and related nitrogenous nutrients in feedstuffs for animal diets. Amino Acids 2020, 1-20, doi:10.1007/s00726-020-02833-4.]. Which translates into FM and CM containing at least 2.2 % and 1.1 % proteins <1000 Da respectively. The capacity of proteinaceous materials to scavenge free radicals depends on the protein molecular weight distribution. Proteins with low molecular weight peptides could scavenge free radicals more efficiently. Free radical scavenging activity of proteinaceous molecules is also influenced by: (1). Amino acid composition: hydrophobic amino acids (for *e.g*. Tyr, Phe, Pro, Ala, His and Leu) have superior radical scavenging activity in comparison to hydrophilic amino acids; (2). Amino acid sequence: Peptides with amphiphilic nature could enhance radical scavenging activity of a sample. Chemical analyses have indicated that Tyr exhibit antioxidant behavior via hydrogen atom transfer mechanism. On the other hand, amino acids such as Cys, Trp and His exhibit antioxidant behavior via single electron transfer mechanism.

FM and CM exhibit pro-oxidant behavior at most concentrations tested after 30 min as well as 60 min of incubation (see Figure 4 and Figure 5). This behavior mainly arises from the thermal processing. For both FM and CM, thermal processing commonly involves heating the raw product at high temperatures for 15 to 20 min. In Norway, during fishmeal production, wild caught fishes are subjected to heating at temperatures ≥70°C for time ≥20 min in order to achieve 100 log₁₀ reductions of *Enterobacteriaceae* and *Salmonella* counts. Such strict thermal processing conditions may result in oxidation of fats and proteins. Fish meal contains lipids rich in polyunsaturated fatty acids that are more susceptible to thermal oxidation. Antioxidant are commonly added in fish meal to prevent the oxidation of polyunsaturated fatty acids (also visible in table 1). Heat induced oxidation of amino acids lead to development of wide range oxidation products. The pro-oxidant behavior of amino acid oxidation by products is already known. They can result in a wide range of health conditions in animal body. All the black soldier fly protein derivatives (puree, hydrolysed puree) used and analyzed by the inventors were thermally processed at temperatures <100°C for time <.1.5 min (*e.g*. at 90°C for 80 seconds). These thermal processing time-temperature combinations of the current invention were adopted to ensure minimum damage to nutrients (proteins and fat) and adequate inactivation of pathogenic microbiota. This implies that pro-oxidant behavior of FM and CM arises mainly due to stringent production method.

In one of the recent studies [3], researchers made BSF protein hydrolysate using bromelain enzyme. Bromelain derived protein hydrolysate was also tested for DPPH radical scavenging activity, which resulted into the IC₅₀ of 8.4 mg/ml. The DPPH radical scavenging activity of this bromelain derived protein hydrolysate was much lower when compared to the activity of products such as BSF-HP (IC₅₀ 0.18 mg/ml after 60 min of incubation). The higher activity of BSF-HP as found by the inventors could arise from compositional attributes (as previously discussed in this section) and quality of raw material itself. Protix is reportedly producing insect proteins in GMP+ and SecureFeed certified facilities, under HACCP conditions.

### 4.3. ABTS cation radical scavening activity

ABTS cation radical scavenging denotes the ability of sample to donate electron and stabilize free radicals. ABTS assay IC₅₀ of all samples are indicated in table 5. They are in following order: FM> CM> BSF-HP> BSF-P. The higher the IC₅₀, the lower the antioxidant activity. In this assay even FM and CM exhibit antioxidant activity. It appears that FM and CM extracts may be efficient where free radical(s) could be stabilized using single electron transfer mechanism. However, they still exhibit lower scavenging activity in comparison to the surprisingly high scavenging activity of BSF derivatives BSF larvae puree and hydrolysed BSF larvae puree.

Dependence of radical scavenging activity on protein molecular weight is already explained in section 4.2. At Protix the inventors established that BSF-P and BSF-HP have exactly the same amino acids composition. However, due to protein hydrolysis, the amount of proteins <1000 Da is higher in BSF-HP than in BSF-P. It is therefore somewhat surprising that the BSF-P IC₅₀ value was slightly lower in comparison to BSF-HP. This could be explained by the mechanism of hydrolysis. Enzymatic hydrolysis is achieved through exo- and endo- peptidase. Exopeptidase cleaves the terminal peptide bond, on the other hand endopeptidase cleaves the non-terminal peptide bond. In both cases the sequence of amino acids is altered. The radical scavenging ability of resulting peptides via single electron transfer is also dependent on the amphiphilic nature of proteinaceous molecules. It is possible that peptides in BSF-HP are less amphiphilic in nature that results into lower ABTS cation radical scavenging activity of BSF-HP compared to BSF-P.

Zhu et al. [4] developed BSF protein hydrolysate using wide range of commercial enzymes. The hydrolysates were further fractionated into following group: group 1 (<3000 Da), group 2 (3000 to 10,000 Da) and group 3 (>10,000 Da) using ultrafiltration. The activity of these hydrolyzed fractionates were also investigated for ABTS cation radical scavenging activity. Ascorbic acid was used as the reference molecule in the example. Interestingly the best performing fractionate and ascorbic acid were able to inhibit 85.67 % and 92.11 % of ABTS cation radical at 0.05 mg/ml concentration, respectively. The current inventors now surprisingly established that BSF-P already exhibits ABTS cation radical scavenging Eₘₐₓ of as high as 89, (at 0.2 mg/ml). This shows that fractioning BSF-P reveals fractions that have very strong antioxidant potential.

### 4.4. Neutrophil response modulation activity

Strong free radical scavenging activities of BSF derivatives are evident from section 4.2 and 4.3. Furthermore, all the samples were also tested for neutrophil response modulation activity. Neutrophils are white blood cells present in animal body (including humans, pets, fishes, poultry and swine). They are involved in the primary defense against pathogens. When pathogenic microbes enter the animal body, neutrophils rush to the site of infestation and initiate defense. During granulation, neutrophil release a wide range of oxidative enzymes including NADPH oxidase, which is responsible for production of superoxide anion and by product (*e.g*. hydrogen peroxide). Superoxide anion can further react with nitric oxide radical to produce peroxynitrite. This process also generates hydroxyl radical (by reaction of hydrogen peroxide with metal ion). This battery of oxidative reactions are crucial to the defense of the host animal. However, these ROS generated during host defense can react with enzymes, proteins, lipids, etc. of body cells and result in the development of different health conditions (for *e.g*. cellular ageing, cancer, *etc*.). The neutrophil assay conducted in this research determines the ability of proteinaceous molecules to scavenge ROS produced as a result of neutrophil activity. PMA was used to activate protein kinase C present in neutrophils, which results in production of NADPH oxidase responsible for catalyzing ROS production. ROS production in system is coupled with lucigenin amplified chemi-luminescence. Ability of proteinaceous sample to scavenge ROS (particularly superoxide anion) is marked by decreased chemi-luminescence.

To the inventors knowledge, this is the first analysis of *in vitro* neutrophil response modulation activity of BSF derivatives BSF larvae puree and hydrolysed BSF larvae puree. CM exhibited pro-oxidant behavior at 3 out of 5 tested concentrations, and Eₘₐₓ of only 5% at 0.2 mg/ml (see figure 9 and table 6). CM is commonly used in pet food preparations. However, the comparative test results in the examples and embodiments of the invention indicate that CM inclusion offers little or no benefits relating to scavenging the ROS produced by neutrophils. Moreover, CM inclusion could even result in inflammatory damage to host cells. Repetitive inflammatory damage of canine or feline cells could translate into conditions such as accelerated aging, slow cognitive function, etc.

On the other hand, FM exhibits mild anti-oxidant behavior in this assay, with Eₘₐₓ of 22% (see table 6). At 0.2 mg/ml, FM exhibits inhibition of 5%. Aquaculture rearing media (*i.e*. water) offers a continuous buffer of pathogenic bacteria. Therefore, aquaculture organisms are at constant risk of pathogenic bacterial invasions. This results in wide range of health conditions, including reduced immunity, aging, *etc.* The comparative examples of the current inventors highlight the inadequacy of FM to suppress the inflammatory damage from repetitive neutrophil activity. This often translates into incremental cost occurring as a result of antibiotics and nutritional supplement usage. BSF-P exhibits Eₘₐₓ and IC₅₀ of 59.57% and 0.15 mg/ml, respectively (see table 6). Moreover, BSF-HP also exhibits neutrophil response modulation activity comparable to BSF-P (see table 5).

### 4.5. MPO response modulation activity (SIEFED and classical assay)

The general mechanism of neutrophil response is known. The neutrophil extracellular trap contains several molecules required to inactivate pathogenic microbes. MPO enzyme present in neutrophil extracellular trap can produce hypochlorous acid from hydrogen peroxide and chloride ion. Additionally, MPO is capable of oxidizing tyrosine into the tyrosyl free radical. Both products of MPO oxidation (hypochlorous acid and tyrosyl free radical) are crucial to inactivate pathogens. Again, repetitive interaction of these molecules with animal cells result in inflammatory damage. In an animal body, MPO-Fe(lll) (active form) reacts with hydrogen peroxide to form oxoferryl π cation radical (Cpl form). Cpl form converts back into MPO-Fe(III) coupled with chloride ion transforming into hypochlorous acid. However, in the present experiment, back reduction of the Cp I form to MPO-Fe(III) was achieved in 2 stages. First reduction of Cpl to MPO-Fe(IV)=O via electron transfer through nitrite ions. Then, electron provisioning was done (via Amplex^{™} Red oxidation to resorufin reaction) which converts MPO-Fe(IV)=O to MPO-Fe(III) form. Proteinaceous molecules could prevent the oxidative damage resulting from MPO either by directly reacting with Cpl form and terminating the halogenation, or by donating hydrogen (hydrogen atom transfer) to ROS produced as a consequence of MPO activity. MPO response modulation activity was analyzed using the classical and SIEFED assay. The classical assay measures ability of sample to complex with Cpl form and stabilize ROS. Whereas in SIEFED assay, MPO is bound to rabbit polyclonal antibodies (and rest of the compounds are washed away), so it purely measures the ability of samples to complex with Cpl form.

As with neutrophil response modulation activity, MPO response modulation activity of BSF derivatives BSF larvae puree and hydrolysed BSF larvae puree is also being established by the inventors for the first time, after provision of the puree according to the method of the invention. FM and CM exhibit pro-oxidant behavior in both the assays (see figure 7 and 8). Presence of oxidative reaction products in FM and CM (as a consequence of production process) that are capable of initiating pro-oxidative response has been already discussed in section 4.2. Detailed *in vitro* investigations realized by the inventors indicate that inclusion of FM and CM in animal diets may result in inflammatory damage.

In classical assay, BSF derivatives exhibit surprisingly strong antioxidant potential, with IC₅₀ in following order: BSF-P> BSF-HP. In the SIEFED assay, BSF-P did not reach 50% inhibition (even at highest concentration tested). Thus while, BSF-P is more effective in stabilizing ROS, BSF-HP has higher efficacy in complexing with Cpl form of MPO. These observations show that the two BSF derivatives are suitable for use as an ingredient in pet food and aquaculture formulations to effectively suppress inflammatory damages resulting from MPO activity.

BSF protein derivatives offer anti-oxidative advantage over FM and CM.

### EXAMPLE 4

Fat oxidation as a consequence of puree heating times

### Introduction

Pasteurization is crucial for production of processed insect protein. Optimal heating time and optimal heating temperature combinations is important for:
(1). inactivation of pathogens; and
(2). preserving the nutritional quality of food.

With respect to fat quality (of insect proteins), pasteurization time x temperature combination is significant for:
(1). inactivation of intestinal SN-1,3- lipase: If not inactivated, this enzyme could hydrolyze triglycerides into free fatty acids (present at SN-1,3 position of triglycerides) and 2-monoglycerides.
(2). Lipid oxidation: Heating could result in the (non-enzymatic) oxidation of polyunsaturated fatty acids. The free fatty acids formed due to lipase activity could also undergo (enzymatic) oxidation via lipoxygenases. These two oxidation processes result in the formation of primary (*e.g.* hydroperoxides) and secondary oxidation products (*e.g*. aldehydes and ketones). These reactions have adverse effect on the sensory quality of food and also the health of consuming animals.

In this current Example 4 the extent of FFA formation in pasteurized puree (to analyze lipase inactivation) and development of hydroperoxides as a consequence of heating times, is established.

### Materials and Methods

Approximately 5 kg live black soldier fly larvae were harvested from a rearing unit (Protix, The Netherlands). These larvae were washed thoroughly to remove all visible impurities. 300 g of washed larvae were used per treatments. Larvae were minced to obtain a smooth slurry (puree) and pasteurized according to treatments indicated in Table 7. The pasteurized larvae puree was immediately frozen (to - 20°C). These frozen samples were later thawed and analysed for free fatty acid content and peroxide values. All the experiments were performed in duplicates.

**Table 7. Description of pasteurization treatments used in Example 4.**

| Treatment | Pasteurization temperature (°C) | Pasteurization time (s) |
|---|---|---|
| 1 | 90 | 0 |
| 2 | 90 | 40 |
| 3 | 90 | 80 |
| 4 | 90 | 300 |
| 5 | 90 | 1800 |

### Results

The free fatty acid contents and peroxide values obtained for respective treatments are mentioned in Table 8.

**Table 8. Free fatty acid and peroxide values obtained for respective treatments**

| Treatment | Pasteurization temperature (°C) | Pasteurization time (s) | Free fatty acid content (%)¹ | Peroxide value (meq/kg) ¹ |
|---|---|---|---|---|
| 1 | 90 | 0 | 1.2 ± 0.5 | 1.1 ± 1.3 |
| 2 | 90 | 40 | 69.8 ± 12.6 | 2.8 ± 0.5 |
| 3 | 90 | 80 | 0.9 ± 0.1 | 1.1 ± 1.0 |
| 4 | 90 | 300 | 1.4 ± 0.4 | 1.3 ± 1.0 |
| 5 | 90 | 1800 | 1.15 ± 0.15 | 1.2 ± 0.0 |

| | | | | |
|---|---|---|---|---|
| ¹Results expressed at mean ± S.D. (n=2) | | | | |

As visible from Table 8, that insect puree heated at 90°C for 40 s has relatively high amounts for free fatty acids (approx. 70%). Heating puree just for 40 s is ineffective in the deactivation of lipase. Whereas, low amounts of free fatty acids are observed when puree is heated for 80 s, indicating the sufficiency of this time for enzyme inactivation at 90°C. At heating times of over 80 seconds (treatment 4 and 5 in Table 7 and Table 8), the fatty acid content increases when compared to the low fatty acid content obtained when the black soldier fly larvae puree is heated for 80 seconds. Therefore, for obtaining a protein fraction which comprises a relatively low free fatty acid content, the heating time in the method of the invention should be longer than 40 seconds and shorter than 300 seconds, such as 50 - 100 seconds, or a heating time selected from 60 - 90 seconds, or 70 - 85 seconds, such as about 80 seconds. Preferred heating time is about 90°C or 90°C ± 2°C.

Also higher peroxide value is observed for heating duration of 40 s. This could be explained by high amounts of free fatty acids that could participate in enzyme oxidation leading to the production of hydroperoxides. However, in case of 80 s treatment, lower peroxide value is obtained. Heating the product further has an effect on fat oxidation in that the peroxide value for Treatment 4 and Treatment 5 is increased compared to the peroxide value for Treatment 3, *i.e.* a heating time of 80 seconds. Therefore, for obtaining a protein fraction which comprises a relatively low peroxide value, the heating time in the method of the invention should be longer than 40 seconds and shorter than 300 seconds, such as 50 - 100 seconds, or a heating time selected from 60 - 90 seconds, or 70 - 85 seconds, such as about 80 seconds. Preferred heating time is about 90°C or 90°C ± 2°C.

### Conclusion

Heating the minced insect larvae at 90°C for 80 s is sufficient for enzyme inactivation, prevents free fatty acid formation, and prevents peroxide formation.

### EXAMPLE 5

### Antioxidant activity of Fish samples after feeding fish with feed supplement

Antioxidant activity of Fish fillet samples was investigated using anti-radical (ABTS) technique. Water soluble black soldier fly larvae protein extracts, referred to as HI-0, HI-25, HI-50 and HI-100, were prepared in distilled water and used at different concentrations. In the ABTS assay, the percentage inhibition at maximum tested concentration was in following order: HI-25 > HI-100 > HI-50 > HI-0. In the ABTS assay, the HI-25 filet sample reached the IC50 ≥50%. In conclusion, within the concentration series tested, the HI-25 sample had the maximum ABTS radical scavenging activity among all the tested Fish fillet samples.

Using the ABTS assay, activity of fish flesh samples was assessed with regard to radical lowering activity of black soldier fly protein obtained with the method of the invention.

### Materials and Methods

### Diets

ProteinX (high protein meal of *Hermetia illucens*) used in this study was produced according to the method of the invention and was provided by Protix (The Netherlands). Two iso-nitrogenous, iso-lipidic and iso-energetic diets where formulated with either fish meal (20.6%) or ProteinX (32%) as the main protein source. Both diets where produced by Research Diet Services (the Netherlands). Diamol was added to both diets as inert marker for digestibility measurements. Four treatments where formed by feeding either one of both feeds, or mixing both feeds to a determined ratio.

The dietary treatments where referred to as:
HI0 (100% fish meal based feed);
HI25 (75% fish meal based feed mixed with 25% ProteinX based feed);
HI50 (both feeds mixed in equal ratio; 50% fish meal based feed mixed with 50% ProteinX based feed); and
HI100 (100% ProteinX based feed).

The proximate composition of all four dietary treatments is presented in Table 9.

The proximate composition of ProteinX and of the four experimental dietary treatments was determined by DISAFA laboratories and is presented in Table 9. Feed samples were ground using a cutting mill (MLI 204; Bühler AG, Uzwil, Switzerland) and analysed for dry matter content (AOAC #934.01), crude protein content (AOAC #984.13), ash content (AOAC #942.05), and ether extract (AOAC #2003.05) were analysed according to AOAC International. Crude protein content was calculated as: *nitrogen* * 6.25

### Fish and rearing conditions

The animal feeding trial was conducted at the Experimental Facility of the Department of Agricultural, Forest, and Food Sciences of the University of Torino (Italy). The experimental protocol was designed according to the guidelines of the current European and Italian laws on the care and use of experimental animals (European directive 86 609/EEC, put into law in Italy with D.L. 116/92). The experimental protocol was approved by the Ethical Committee of the University of Turin (protocol n° 143811). Juvenile rainbow trout were obtained from a private hatchery (Troticoltura Bassignana, Cuneo, Italy) and randomly distributed over 12 fiberglass tanks (*n* = 3) (50 fish per tank). Tanks were supplied artesian well water (13 ±1°C) in a flow-through system at a flow rate of 8 L/min. Dissolved oxygen was measured every fortnight and ranged between 7.6 and 8.7 mg / L. Fish were acclimatized to the experimental set up for four weeks during which a commercial diet was fed at 1.6% of the total body weight per tank. After acclimatization, fish were restrictively fed a dietary treatment for 133 days of which 113 days at a fixed ratio of 1.4% followed by 20 days at a fixed ratio of 1.1%. Feed intake was monitored at each administration. Feed was distributed by hand twice a day, 6 days per week. The biomass of each tank was weighed in bulk every 14 days to correct the daily feeding rate. Mortality was checked daily.

| **Table 9**. Formulation and proximate composition (analysis on wet-weight basis) of the four experimental dietary treatments fed to rainbow trout (*Oncorhynchus mykiss*) and proximate composition of ProteinX. | | | | | |
|---|---|---|---|---|---|
| | **HI0** | **HI25** | **HI50** | **HI100** | **ProteinX** |
| Ingredients (g/kg) | | | | | |
| Fish meal | 206 | 154,5 | 103 | 0 | |
| Soybean protein concentrate | 150 | 150 | 150 | 150 | |
| Wheat gluten meal | 100 | 100 | 100 | 100 | |
| Corn gluten meal | 70 | 70 | 70 | 70 | |
| Soybean meal | 40 | 40 | 40 | 40 | |
| Wheat meal | 240.5 | 218.225 | 195.95 | 151.4 | |
| Protein X | 0 | 80 | 160 | 320 | |
| Fish oil | 50 | 50 | 50 | 50 | |
| Soybean oil | 123.5 | 111.375 | 99.25 | 75 | |
| Vit. min. premix (1%) | 10 | 10 | 10 | 10 | |
| *DL methionine* | | 0.275 | 0.55 | 1.1 | |
| *L-lysine HCL* | | 0.6 | 1.2 | 2.4 | |
| Diamol | 10 | 10 | 10 | 10 | |
| Lime fine | | 1.62 | 3.25 | 6.5 | |
| Monocalcium phosphate | | 2 | 4 | 8 | |
| Salt | | 1.625 | 3.25 | 5 | |
| Magnesium oxide | | 0.15 | 0.3 | 0.6 | |

| Proximate analysis | | | | | |
|---|---|---|---|---|---|
| DM (%) | 93.93 | 94.81 | 94.41 | 96.01 | 96.24 |
| Crude Protein (%) | 41.10 | 41.73 | 42.33 | 44.05 | 55.35 |
| Crude Lipid (%) | 18.85 | 19.09 | 19.32 | 19.79 | 19.67 |
| Ash (%) | 5.43 | 6.31 | 6.11 | 6.44 | 5.35 |

### Sampling:

At the end of the trial, fish were fasted for one day, and 9 fish/treatment (3 fish/tank) fish per treatment where killed by overdose of anaesthesia (MS-222 - PHARMAQ Ltd, UK; 500 mg/L) and individually weighed. The right fillets were collected of each fish and vacuum-sealed, frozen (-20°) and subsequently freeze dried.

### Sample preparation:

Fish samples (in powdered form) were received. The samples were kept in a freezer (-20°C) until further processing. Water soluble extract powders (WSEP) of respective samples were made according to the protocol of Mouithys-Mickalad *et al.* (2020). WSEP were stored in a desiccator (at 18°C) until further experimentations. The stock solutions of the WSEP were made by dissolving to a final concentration of 20 mg/mL in Milli-Q water, which stock solution will be then diluted by half to obtain 10, 5, 2.5 and 1.25 mg/mL, successively.

### ABTS radical scavenging activity:

The radical scavenging activity of WSEP samples toward ABTS radical cation were analysed according to the method of Arnao et al. (2001). ABTS stock solution was made by completely dissolving 7.0 mmol/L ABTS and 1.35 mmol/L potassium persulfate in Milli-Q water. The test solution was kept overnight in dark (at 18°C) for reaction to complete. ABTS working solution was made by diluting stock solution with methyl alcohol to obtain absorbance between 0.7 to 0.8 mm at 734 nm. ABTS working solution (1920 µL) was mixed with 20 µL of WSEP solutions (obtained by dissolving WSEP in Milli-Q water) to obtain a final concentration of 0.0125, 0.025, 0.05, 0.1 and 0.2 mg/ml. Decrease in absorbance post 30 min of incubation in dark was measured at 734 nm using an HP 8453 UV-vis spectrophotometer. Instead of WSEP dilution, only Milli-Q water was used in case of control. The repetitions with increasing concentrations of different samples was performed as described by Paul (2007) and transferred into a multi-well device (96-well plate) with a final volume of 200 µL (198 µL of solvent and 2 µL of WSEP). Likewise, after a 30-min incubation period, the absorbance was read at 740 nm using a Multiskan Ascent reader (Thermo Fisher Scientific).

### Results

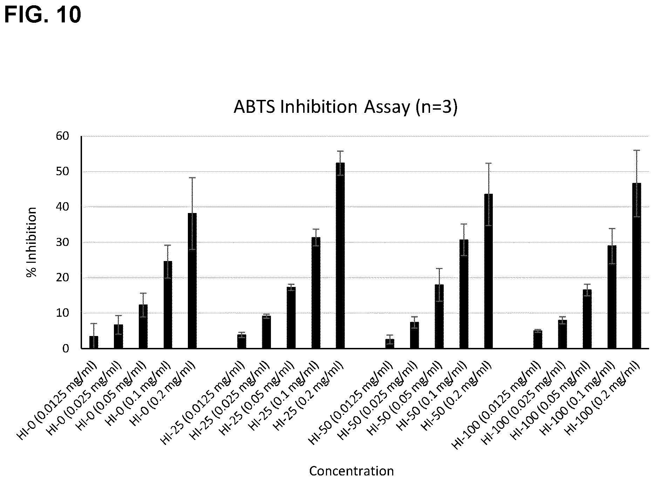

ABTS radical scavenging activity of WSEP samples is indicated in Figure 10: displayed is the ABTS radical scavenging activity of WSEP performed in methanol. Results are mean ± SD of three independent assays in triplicate. For all the samples increase in concentration resulted into an increase of mean inhibition values (indicating anti-oxidant behaviour). Eₘₐₓ (maximum inhibition obtained during the test) were in the following order: HI-25 > HI-100 > HI-50 > HI-0 (all obtained at 0.2 mg/ml, final concentration). This result indicates that HI-25 samples exhibit highest ABTS radical scavenging activity. On the other hand, HI-0 samples exhibit least ABTS radical scavenging activity. Eventually, HI-25 samples exhibited mean inhibition values ≥ 50% (IC₅₀ was achieved).

### Concluding remark

As assessed by using the ABTS assay, all the protein samples obtained by applying the method of the invention with black soldier fly larvae, exhibit anti-oxidant activity in a dose-dependent manner. HI-25 samples show the highest anti-oxidant activity.

### References

1. Tsumbu, C.N.; Deby-Dupont, G.; Tits, M.; Angenot, L.; Frederich, M.; Kohnen, S.; Mouithys-Mickalad, A.; Serteyn, D.; Franck, T. Polyphenol content and modulatory activities of some tropical dietary plant extracts on the oxidant activities of neutrophils and myeloperoxidase. Int J Mol Sci 2012, 13, 628-650, doi:10.3390/ijms13010628.
2. Paul, A. Field border flowering strips as a source of valuable compounds, Gembloux Agro-Bio Tech University of Liège, Gembloux, Belgique, 2017.
3. Firmansyah, M.; Abduh, M.Y. Production of protein hydrolysate containing antioxidant activity from Hermetia illucens. Heliyon 2019, 5, e02005, doi:10.1016/j.heliyon.2019.e02005.
4. Zhu, D.; Huang, X.; Tu, F.; Wang, C.; Yang, F. Preparation, antioxidant activity evaluation, and identification of antioxidant peptide from black soldier fly (Hermetia illucens L.) larvae. J. Food Biochem. 2020, e13186, doi:10.1111/jfbc.13186.
5. Smith, P.K.; Krohn, R.I.; Hermanson, G.T.; Mallia, A.K.; Gartner, F.H.; Provenzano, M.D.; Fujimoto, E.K.; Goeke, N.M.; Olson, B.J.; Klenk, D.C. Measurement of protein using bicinchoninic acid. Anal. Biochem. 1985, 150, 76-85, doi:10.1016/0003-2697(85)90442-7.
6. Brand-Williams, W.; Cuvelier, M.E.; Berset, C. Use of a free radical method to evaluate antioxidant activity. LWT - Food Science and Technology 1995, 28, 25-30, doi:10.1016/S0023-6438(95)80008-5.
7. Arnao, M.B.; Cano, A.; Acosta, M. The hydrophilic and lipophilic contribution to total antioxidant activity. Food Chemistry 2001, 73, 239-244, doi:10.1016/S0308-8146(00)00324-1.
8. Franck, T.; Kohnen, S.; Boudjeltia, K.Z.; Van Antwerpen, P.; Bosseloir, A.; Niesten, A.; Gach, O.; Nys, M.; Deby-Dupont, G.; Serteyn, D. A new easy method for specific measurement of active myeloperoxidase in human biological fluids and tissue extracts. Talanta 2009, 80, 723-729, doi:10.1016/j.talanta.2009.07.052.
9. Zhang, H.; Holden-Wiltse, J.; Wang, J.; Liang, H. A Strategy to Model Nonmonotonic Dose-Response Curve and Estimate IC50. PLOS ONE 2013, 8, e69301, doi:10.1371/journal.pone.0069301.
10. R Core Team R: A language and environment for statistical computing; R Foundation for Statistical Computing: Vienna, Austria, 2017.
11. Mouithys-Mickalad, A., Schmitt, E., Dalim, M., Franck, T., Tome, N.M., van Spankeren, M., Serteyn, D., Paul, A., 2020. Black Soldier Fly (Hermetia illucens) Larvae Protein Derivatives: Potential to Promote Animal Health. Animals 10, 941. https://doi.org/10.3390/ani10060941

## Claims

1. Method to convert insects into nutrient streams, comprising the steps of:
a) providing fresh insects and preparing a pulp thereof;
b) heating the pulp for 50-100 seconds at a temperature of 60°C-95°C.

2. Method according to claim 1, further comprising a step a1) of treating the pulp by an enzyme prior to the step b).

3. Method according to claim 1, wherein the pulp is not enzymatically treated prior to heating.

4. Method according to any of the claims 1-3, comprising a further step c) after step b), wherein the heated pulp or the heated and hydrolysed pulp is subjected to a physical separation step thereby obtaining a fat fraction, an aqueous protein fraction and a solid-containing fraction.

5. Method according to claim 4, comprising a further step of drying the protein fraction revealing a protein meal.

6. A method according to claim 4, comprising a further step of combining both the solid fraction and the aqueous protein fraction, and drying to produce a dried material.

7. A method according to any of the claims 1-3, comprising a further step of separating the fat fraction and subjecting the remaining mixture to drying to produce a dried material.

8. A pulp obtainable by the method according to claim 1.

9. A hydrolysed pulp obtainable by the method according to claim 2.

10. A fat fraction obtainable by the method according to claim 4.

11. A aqueous protein fraction obtainable by the method according to claim 4.

12. A solid-containing fraction obtainable by the method according to claim 4.

13. A protein meal obtainable by the method according to claim 5.

14. A dried material obtainable by the method according to claim 6 or 7.

15. Use of any one or more of:
- the pulp of claim 8,
- the hydrolysed pulp of claim 9
- the fat fraction of claim 10,
- the aqueous protein fraction of claim 11,
- the solid-containing fraction of claim 12,
- the protein meal of claim 13, and
- the dried material of claim 14,
as a food product, feed product, feed or feed ingredient.
